# EUROPEAN PATENT APPLICATION

(11) **EP 2 992 875 A1**
(43) Date of publication of application: **09.03.2016**
(21) Application number: 15161527.5
(22) Date of filing: 20.04.2007
(51) Int. Cl.: A61K 9/127, A61K 47/48

(54) **LIPOPLEX FORMULATIONS FOR SPECIFIC DELIVERY TO VASCULAR ENDOTHELIUM**

(30) Priority: 20.04.2006 EP 06008209
(62) Divisional of application: 07724432.5
(71) Applicant: Silence Therapeutics GmbH, 13125 Berlin (DE)
(72) Inventor: Kaufmann, Jorg, 13465 Berlin (DE); Keil, Oliver, 13467 Berlin (DE); Santel, Ansgar, 10779 Berlin (DE)
(74) Representative: Stratagem IPM Limited

(57) **Abstract**

The present invention is related to a lipid composition contained in and/or containing a carrier comprising at least a first lipid component, at least a first helper lipid, and a shielding compound which is optionally removable from the lipid composition under in vivo conditions, whereby the lipid composition containing carrier has an osmolality of about 50 to 600 mosmole/kg, preferably about 250 - 350 mosmole/kg, and more preferably about 280 to 320 mosmole/kg, and/or whereby liposomes formed by the first lipid component and/or one or both of the helper lipid and the shielding compound in the carrier have a particle size of about 20 to 200 nm, preferably about 30 to 100 nm, and more preferably about 40 to 80 nm.

SEQUENCE LISTING

<110> Silence Therapetics GmbH

<120> Lipoplex Formulations

<130> P6246

<150> EP06008209.6
<151> 2006-04-20
<150> PCT/EP2007/003496
<151> 2007-04-20

<160> 23

<170> BiSSAP 1.2

<210> 1
<211> 19
<212> RNA
<213> Artificial Sequence

<220>
<221> source
<222> 1..19
<223> /organism="Artificial Sequence"
      /note="PKN3 sense"
      /mol_type="unassigned RNA"

<220>
<221> misc_feature
<222> 2,4,6,8,10,12,14,16,18
<223> /note="2'-O-methyl modified"

<400> 1
gagagccugu acugcgaga                                                    19


<210> 2
<211> 19
<212> RNA
<213> Artificial Sequence

<220>
<221> source
<222> 1..19
<223> /organism="Artificial Sequence"
      /note="PKN3 antisense"
      /mol_type="unassigned RNA"

<220>
<221> misc_feature
<222> 1,3,5,7,9,11,13,15,17,19
<223> /note="2'-O-methyl modified"

<400> 2
ucucgcagua caggcucuc                                                    19


<210> 3
<211> 19
<212> RNA
<213> Artificial Sequence

<220>
<221> source
<222> 1..19
<223> /organism="Artificial Sequence"
      /note="PTEN sense"
      /mol_type="unassigned RNA"

<220>
<221> misc_feature
<222> 2,4,6,8,10,12,14,16,18
<223> /note="2'-O-methyl modified"

<400> 3
ccaccacagc uagaacuua                                                    19


<210> 4
<211> 19
<212> RNA
<213> Artificial Sequence

<220>
<221> source
<222> 1..19
<223> /organism="Artificial Sequence"
      /note="PTEN antisense"
      /mol_type="unassigned RNA"

<220>
<221> misc_feature
<222> 1,3,5,7,9,11,13,15,17,19
<223> /note="2'-O-methyl modified"

<400> 4
uaaguucuag cuguggugg                                                    19


<210> 5
<211> 19
<212> RNA
<213> Artificial Sequence

<220>
<221> source
<222> 1..19
<223> /organism="Artificial Sequence"
      /note="CD31 sense"
      /mol_type="unassigned RNA"

<220>
<221> misc_feature
<222> 2,4,6,8,10,12,14,16,18
<223> /note="2'-O-methyl modified"

<400> 5
uuccguucua gaguaucug                                                    19


<210> 6
<211> 19
<212> RNA
<213> Artificial Sequence

<220>
<221> source
<222> 1..19
<223> /organism="Artificial Sequence"
      /note="CD31 antisense"
      /mol_type="unassigned RNA"

<220>
<221> misc_feature
<222> 1,3,5,7,9,11,13,15,17,19
<223> /note="2'-O-methyl modified"

<400> 6
cagauacucu agaacggaa                                                    19


<210> 7
<211> 19
<212> RNA
<213> Artificial Sequence

<220>
<221> source
<222> 1..19
<223> /organism="Artificial Sequence"
      /note="PTEN sense"
      /mol_type="unassigned RNA"

<220>
<221> misc_feature
<222> 2,4,6,8,10,12,14,16,18
<223> /note="2'-O-methyl modified"

<400> 7
ccaccacagc uagaacuua                                                    19


<210> 8
<211> 19
<212> RNA
<213> Artificial Sequence

<220>
<221> source
<222> 1..19
<223> /organism="Artificial Sequence"
      /note="PTEN antisense-Cy3"
      /mol_type="unassigned RNA"

<220>
<221> misc_feature
<222> 1,3,5,7,9,11,13,15,17,19
<223> /note="2'-O-methyl modified"

<220>
<221> misc_feature
<222> 19
<223> /note="Cy3 label"

<400> 8
   uaaguucuag cuguggugg                                                   19

<210> 9
<211> 19
<212> RNA
<213> Artificial Sequence

<220>
<221> source
<222> 1..19
<223> /organism="Artificial Sequence"
      /note="PTEN sense"
      /mol_type="unassigned RNA"

<220>
<221> misc_feature
<222> 2,4,6,8,10,12,14,16,18
<223> /note="2'-O-methyl modified"

<400> 9
ccaccacagc uagaacuua                                                    19


<210> 10
<211> 19
<212> RNA
<213> Artificial Sequence

<220>
<221> source
<222> 1..19
<223> /organism="Artificial Sequence"
      /note="PTEN antisense-Cy5"
      /mol_type="unassigned RNA"

<220>
<221> misc_feature
<222> 1,3,5,7,9,11,13,15,17,19
<223> /note="2'-O-methyl modified"

<220>
<221> misc_feature
<222> 19
<223> /note=" Cy5 label"

<400> 10
uaaguucuag cuguggugg                                                    19


<210> 11
<211> 19
<212> RNA
<213> Artificial Sequence

<220>
<221> source
<222> 1..19
<223> /organism="Artificial Sequence"
      /note="Luciferase sense"
      /mol_type="unassigned RNA"

<220>
<221> misc_feature
<222> 2,4,6,8,10,12,14,16,18

<223> /note="2'-O-methyl modified"

<400> 11
cguacgcgga auacuucga                                                    19


<210> 12
<211> 19
<212> RNA
<213> Artificial Sequence

<220>
<221> source
<222> 1..19
<223> /organism="Artificial Sequence"
      /note="Luciferase antisense"
      /mol_type="unassigned RNA"

<220>
<221> misc_feature
<222> 1,3,5,7,9,11,13,15,17,19
<223> /note="2'-O-methyl modified"

<400> 12
ucgaaguauu ccgcguacg                                                    19


<210> 13
<211> 19
<212> RNA
<213> Artificial Sequence

<220>
<221> source
<222> 1..19
<223> /organism="Artificial Sequence"
      /note="Tie2 sense"
      /mol_type="unassigned RNA"

<220>
<221> misc_feature
<222> 2,4,6,8,10,12,14,16,18
<223> /note="2'-O-methyl modified"

<400> 13
auaucugggc aaaugaugg                                                    19


<210> 14
<211> 19
<212> RNA
<213> Artificial Sequence

<220>
<221> source
<222> 1..19
<223> /organism="Artificial Sequence"
      /note="Tie2 antisense"
      /mol_type="unassigned RNA"

<220>
<221> misc_feature
<222> 1,3,5,7,9,11,13,15,17,19

<223> /note="2'-O-methyl modified"

<400> 14
ccaucauuug cccagauau                                                    19


<210> 15
<211> 21
<212> DNA
<213> Artificial Sequence

<220>
<221> source
<222> 1..21
<223> /organism="Artificial Sequence"
      /note="PCR Primer"
      /mol_type="unassigned DNA"

<400> 15
gggaacgaga gccacagaga c                                                 21


<210> 16
<211> 21
<212> DNA
<213> Artificial Sequence

<220>
<221> source
<222> 1..21
<223> /organism="Artificial Sequence"
      /note="PCR Primer"
      /mol_type="unassigned DNA"

<400> 16
cattaaggga gccttccgtt c                                                 21


<210> 17
<211> 28
<212> DNA
<213> Artificial Sequence

<220>
<221> source
<222> 1..28
<223> /organism="Artificial Sequence"
      /note="PCR Primer"
      /mol_type="unassigned DNA"

<220>
<221> misc_feature
<222> 1
<223> /note="-FAM label"

<220>
<221> misc_feature
<222> 28
<223> /note="-TAMRA label"

<400> 17
cggaaggtcg accctaatct catggaaa                                          28

<210> 18
<211> 21
<212> DNA
<213> Artificial Sequence

<220>
<221> source
<222> 1..21
<223> /organism="Artificial Sequence"
      /note="PCR Primer"
      /mol_type="unassigned DNA"

<400> 18
gaggctgatg ctggtgctag t                                                21


<210> 19
<211> 22
<212> DNA
<213> Artificial Sequence

<220>
<221> source
<222> 1..22
<223> /organism="Artificial Sequence"
      /note="PCR Primer"
      /mol_type="unassigned DNA"

<400> 19
cagcaaacac tcaggcctaa cc                                               22


<210> 20
<211> 27
<212> DNA
<213> Artificial Sequence

<220>
<221> source
<222> 1..27
<223> /organism="Artificial Sequence"
      /note="PCR Primer"
      /mol_type="unassigned DNA"

<220>
<221> misc_feature
<222> 27
<223> /note="TAMRA label"

<400> 20
ctgctccctg cttctagccc agtctga                                          27


<210> 21
<211> 21
<212> DNA
<213> Artificial Sequence

<220>
<221> source
<222> 1..21
<223> /organism="Artificial Sequence"

      /note="PCR Primer"
      /mol_type="unassigned DNA"

<400> 21
atgcctctgc tctcaaggat g                                                 21


<210> 22
<211> 22
<212> DNA
<213> Artificial Sequence

<220>
<221> source
<222> 1..22
<223> /organism="Artificial Sequence"
      /note="PCR Primer"
      /mol_type="unassigned DNA"

<400> 22
tctggcaaat cctctatctg tg                                                22


<210> 23
<211> 28
<212> DNA
<213> Artificial Sequence

<220>
<221> source
<222> 1..28
<223> /organism="Artificial Sequence"
      /note="PCR Primer"
      /mol_type="unassigned DNA"

<220>
<221> misc_feature
<222> 1
<223> /note="FAM label"

<220>
<221> misc_feature
<222> 28
<223> /note="-BHQ1"

<400> 23
tgagaaagaa ggcaggccaa ggatgact                                          28

## Description

The present invention is related to a lipid composition and the use thereof.

Although the delivery of pharmaceutically active compounds has already attracted considerable attention in the past, with the advent of new therapeutic agents such as antisense-oligonucleotides and siRNA molecules which are active within a cell, the need for intracellular delivery has become more important and various strategies have been pursued insofar.

In a first step towards the development of siRNA therapeutics, the stability of siRNA molecules was increased by incorporating different chemical backbone modifications preventing these molecules from degradation by nucleases (Chiu and Rana, 2003; Czauderna et al., 2003; Morrissey et al., 2005; Soutschek et al., 2004). For example, blunt-ended siRNA molecules were developed with alternating 2'-O-methyl modification patterns on both strands, which are significantly more resistant to plasma derived nucleases than unmodified siRNAs, and are just as much capable of gene expression silencing in mammalian cells after transfection (Czauderna et al., 2003). Above and beyond the stability issue, functional delivery of siRNA molecules *in vivo* is still the major obstacle and a prerequisite for the development of siRNA therapeutics (Uprichard, 2005). An effective, non-toxic systemic application of siRNA-based therapeutics has not yet been developed for clinical application due to the absence of appropriate non-viral delivery technologies.

A successful delivery technology particularly in case of intracellularly active agents such as siRNA needs to address several problems including the functional intracellular uptake of the highly negatively charged siRNAs, the pharmacodynamic properties for organ and cell type specific *in vivo* delivery, and finally the potential toxic side effects through formulation of siRNA. One strategy extensively tested for *in vivo* delivery of antisense oligonucleotides to overcome these problems, is the direct chemical alteration of the nucleic acid. These chemical modifications are either backbone modifications (Morrissey et al., 2005) or conjugates with uptake mediating entities such as transporting peptides (Muratovska and Eccles, 2004; Richard et al., 2003; Shadidi and Sioud, 2003; Turner et al., 2005) or hydrophobic residues (e.g. cholesterol (Soutschek et al., 2004)). Several reagents have been employed for systemic i.v. administration of siRNAs in mice as an alternative to the covalent modifications of siRNAs. Positively charged carriers, such as protamine-antibody fusion proteins, nanoparticles, cyclodextrin-containing polycation, PEI and atelocollagen have been tested for complex formation with siRNAs for applications *in vivo* (Chae et al., 2004; Hu-Lieskovan et al., 2005; Landen et al., 2005; Schiffelers et al., 2004; Song et al., 2005; Takeshita et al., 2005; Urban-Klein et al., 2005). In order to achieve more cell type specific delivery of siRNAs, some groups have incorporated or conjugated receptor-ligands (Hu-Lieskovan et al., 2005) or antibodies for cellular targeting (Song et al., 2005).

Cationic lipids are routinely used for delivery of nucleic acids into mammalian cells *in vitro* (Felgner et al., 1987), and systemic i.v. administration of lipoplexes (composed of cationic lipid, neutral helper lipid and nucleic acid) have been applied for gene and siRNA delivery *in vivo* (Barron et al., 1999; Chae et al., 2004; Chien et al., 2005; Liu et al., 2004; Morrissey et al., 2005; Nogawa et al., 2005; Yano et al., 2004). However, most of the previous studies were designed for proof of concept but do not provide information on the pharmacodynamic behaviour and possible toxicity of the applied siRNA formulation. This information is a prerequisite for further clinical development with regards to a right risk-benefit-assessment of the particular delivery technology. For example, bioavailability, cell type specific delivery and *in vivo* pharmacokinetics (depending on the routes of administration; i.v., subcutan, oral) are important parameters to evaluate in addition to the demonstration of *in vivo* gene silencing efficacy. While a large number of previous studies report the *in vivo* properties of various modified antisense oligonucelotides, only little is known about the pharmacodynamic behaviour of non-formulated or formulated siRNA and its chemically modified analogues *in vivo.* Intravenous injection of chemically modified siRNAs resulted in a broad tissue distribution with accumulation preferentially in the liver, jejunum and kidney (Braasch et al., 2004; Soutschek et al., 2004). In another study, radioactively labeled siRNA was predominantly detected in muscle and tumor tissue of xenografted mice, and little in liver and kidney when administered i.p. as PEI complex (Urban-Klein et al., 2005). However, these studies did not discriminate the delivery to the many different cell types present in each organ.

The problem underlying the present invention is to provide for a delivery agent for functional nucleic acids such as, but not limited to, siRNA. Furthermore, the problem underlying the present invention is to provide for a delivery agent for functional nucleic acids such as, but not limited to, siRNA, whereby the delivery is specific to endothelium, and more particularly specific for vascular endothelium.

These and other problems are solved by the subject matter of the present invention. More preferred embodiments may be taken from the dependent claims.

The problem underlying the present invention is solved in a first aspect by a lipid composition contained in and/or containing a carrier comprising
at least a first lipid component,
at least a first helper lipid, and
a shielding compound which is optionally removable from the lipid composition under in vivo conditions,
whereby the lipid composition containing carrier has an osmolarity of about 50 to 600 mosmole/kg, preferably about 250 - 350 mosmole/kg, and more preferably about 280 to 320 mosmole/kg, and/or
whereby liposomes formed by the first lipid component and/or one or both of the helper lipid and the shielding compound in the carrier have a particle size of about 20 to 200 nm, preferably about 30 to 100 nm, and more preferably about 40 to 80 nm.

In an embodiment of the first aspect of the present invention the shielding compound is selected from the group comprising PEG, HEG, polyhydroxyethyl starch (polyHES) and polypropylene.

In an embodiment of the first aspect of the present invention the shielding compound is PEG2000 or PEG5000.

In an embodiment of the first aspect of the present invention the first aspect of the present invention the composition comprises a further constituent and/or a second helper lipid.

In an embodiment of the first aspect of the present invention the shielding compound is a conjugate of PEG and ceramide.

In a preferred embodiment of the first aspect of the present invention the ceramide comprises at least one short carbon chain substituent of from 6 to 10 carbon atoms, preferably of 8 carbon atoms.

In an embodiment of the first aspect of the present invention the ceramide is the first helper lipid.

In an alternative embodiment of the first aspect of the present invention the ceramide is the second helper lipid.

In an embodiment of the first aspect of the present invention the shielding compound comprises a pH-sensitive linker or a pH-sensitive moiety.

In a preferred embodiment of the first aspect of the present invention the linker or moiety is an anionic linker or an anionic moiety.

In a more preferred embodiment of the first aspect of the present invention the anionic linker or anionic moiety is less anionic or neutral in an acidic environment, whereby preferably such acidic environment is an endosome.

In an embodiment of the first aspect of the present invention the pH-sensitive linker or the pH-sensitive moiety is selected from the group comprising oligo (glutamic acid), oligophenolate(s) and diethylene triamine penta acetic acid.

In an embodiment of the first aspect of the present invention the first lipid component is a compound according to formula (I), 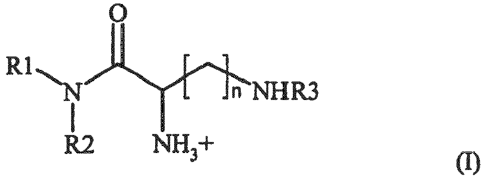 wherein
R₁ and R₂ are each and independently selected from the group comprising alkyl;
n is any integer between 1 and 4;
R₃ is an acyl selected from the group comprising lysyl, ornithyl, 2,4-diaminobutyryl, histidyl and an acyl moiety according to formula (II), 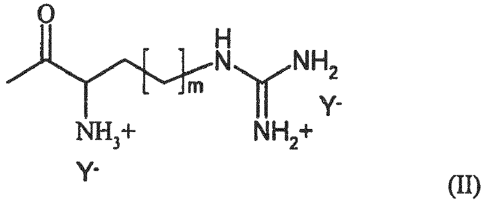
wherein m is any integer from 1 to 3, wherein the NH₃⁺ is optionally absent, and
Y⁻ is a pharmaceutically acceptable anion.

In a preferred embodiment of the first aspect of the present invention R₁ and R₂ are each and independently selected from the group comprising lauryl, myristyl, palmityl and oleyl.

In an embodiment of the first aspect of the present invention R₁ is lauryl and R₂ is myristyl; or
R₁ is palmityl and R₂ is oleyl.

In an embodiment of the first aspect of the present invention m is 1 or 2.

In an embodiment of the first aspect of the present invention the compound is a cationic lipid, preferably in association with an anion Y⁻.

In an embodiment of the first aspect of the present invention Y⁻ is selected from the group comprising halogenids, acetate and trifluoroacetate.

In an embodiment of the first aspect of the present invention the compound is selected from the group comprising
- β-arginyl-2,3-diamino propionic acid-N-palmityl-N-oleyl-amide trihydrochloride 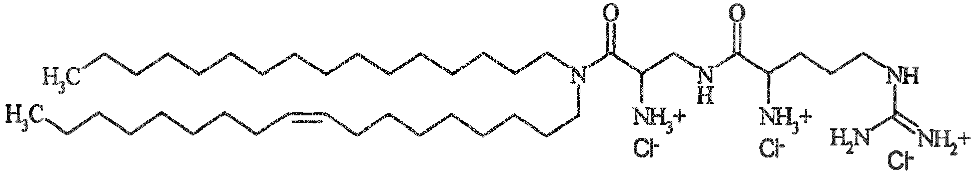
- β-arginyl-2,3-diamino propionic acid-N-lauryl-N-myristyl-amide trihydrochloride 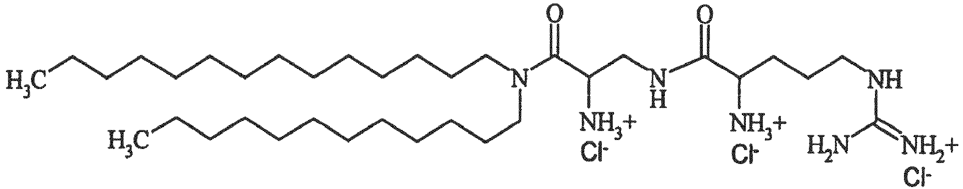 and
- ε-arginyl-lysine-N-lauryl-N-myristyl-amide trihydrochloride

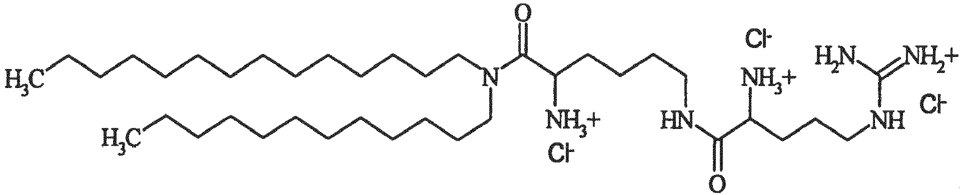

In an embodiment of the first aspect of the present invention the first aspect of the present invention the osmolarity is mostly determined by a sugar, whereby said sugar is preferably selected from the group comprising sucrose, trehalose, glucose, galactose, mannose, maltose, lactulose, inulin, raffinose, and any combination thereof, more preferably selected from the group comprising sucrose, trehalose, inulin, raffinose and any combination thereof.

In an embodiment of the first aspect of the present invention the first aspect of the present invention the composition contains one or several basic compounds, whereby such basic compounds are preferably selected from the group comprising basic amino acids and weak bases.

In an embodiment of the first aspect of the present invention the amino acid is selected from the group comprising histidine, lysine, and arginine.

In an alternative embodiment of the first aspect of the present invention the weak base is selected from the group comprising TRIS and ethanolamine.

In an embodiment of the first aspect of the present invention the basic compound provide for the pH adjustment.

In an embodiment of the first aspect of the present invention the lipid composition comprises a nucleic acid, whereby such nucleic acid is preferably the further constituent.

In a preferred embodiment of the first aspect of the present invention the nucleic acid is selected from the group comprising RNAi, siRNA, siNA, antisense nucleic acid, ribozymes, aptamers and spiegelmers.

In an embodiment of the first aspect of the present invention the shielding compound is attached to the nucleic acid.

In a preferred embodiment of the first aspect of the present invention the shielding compound is attached to the nucleic acid by a linker moiety preferably covalently attached to the nucleic acid by a linker moiety.

In a preferred embodiment of the first aspect of the present invention the linker moiety is selected from the group comprising ssRNA, ssDNA, dsRNA, dsDNA, peptide, S-S-linkers and pH sensitive linkers.

In an embodiment of the first aspect of the present invention the nucleic acid is selected from the group comprising RNAi, siRNA and siNA and the linker is attached to the 3' end of the sense strand.

In an embodiment of the first aspect of the present invention the composition comprises a nucleic acid and the nucleic acid forms together with a/the liposome a lipoplex.

In an embodiment of the first aspect of the present invention the concentration of the lipids in the carrier is about from 0.01 to 100 mg/ml, preferably about from 0.01 to 40 mg/ml and more preferably about from 0.01 to 25 mg/ml, each based on the overall amount of lipid provided by the lipoplex.

In an embodiment of the first aspect of the present invention the nucleic acid is an siRNA and the concentration of the siRNA in the lipid composition is about 0.2 to 0.4 mg/ml, preferably 0.28 mg/ml, and the total lipid concentration is about 1.5 to 2.7 mg/ml, preferably 2.17 mg/ml.

The problem underlying the present invention is solved in a second aspect by a pharmaceutical composition comprising a composition according to the first aspect of the present invention and optionally a pharmaceutically active compound and preferably a pharmaceutically acceptable carrier.

In an embodiment of the second aspect of the present invention the pharmaceutically active compound and/or the further constituent is selected from the group comprising peptides, proteins, oligonucleotides, polynucleotides and nucleic acids.

In an embodiment of the second aspect of the present invention the protein is an antibody, preferably a monoclonal antibody.

In an embodiment of the second aspect of the present invention the nucleic acid is selected from the group comprising DNA, RNA, PNA and LNA.

In an embodiment of the second aspect of the present invention the nucleic acid is a functional nucleic acid, whereby preferably the functional nucleic acid is selected from the group comprising RNAi, siRNA, siNA, antisense nucleic acid, ribozymes, aptamers and spiegelmers.

In an embodiment of any of the first and the second aspect of the present invention the first helper lipid and/or the second helper lipid is selected from the group comprising phospholipids and steroids, preferably under the proviso that the first and/or the second helper lipid is different from a ceramide.

In an embodiment of the first and the second aspect of the present invention the first and/or second helper lipid or helper lipid component is selected from the group comprising 1,2-diphytanoyl-sn-glycero-3-phosphoethanolamine and 1,2-dioleyl-sn-glycero-3-phosphoethanolamine.

In an embodiment of the first and the second aspect of the present invention the content of the helper lipid component is from about 20 mol % to about 80 mol % of the overall lipid content of the composition or of the lipoplex.

In an embodiment of the first and the second aspect of the present invention the content of the helper lipid component is from about 35 mol % to about 65 mol %.

In an embodiment of the first and the second aspect of the present invention the lipid is β-arginyl-2,3-diamino propionic acid-N-palmityl-N-oleyl-amide trihydrochloride, and the helper lipid is 1,2-diphytanoyl-sn-glycero-3-phosphoethanolamine.

In an embodiment of the first and the second aspect of the present invention the lipid is 50 mol% and the helper lipid is 50 mol% of the overall lipid content of the composition or of the lipoplex.

In an embodiment of any of the first and the second aspect of the present invention the composition further comprises a second helper lipid.

In an embodiment of any of the first and the second aspect of the present invention the first and/or the second helper lipid comprises a group which is selected from the group comprising a PEG moiety, a HEG moiety, a polyhydroxyethyl starch (polyHES) moiety and a polypropylene moiety, whereby such moiety preferably provides a molecule weight from about 500 to 10000 Da, more preferably from about 2000 to 5000 Da.

In a preferred embodiment of the first and the second aspect of the present invention the helper lipid comprising the PEG moiety is selected from the group comprising 1,2-distearoyl-sn-glycero-3-phosphoethanolamine and 1,2-dialkyl-sn-glycero-3-phosphoethanolamine.

In an embodiment of the first and the second aspect of the present invention the PEG moiety of the helper lipid has a molecular weight from 2,000 to 5,000 Da, preferably a molecular weight of 2,000 Da.

In a preferred embodiment of the first and the second aspect of the present invention the composition comprises as the lipid component β-arginyl-2,3-diamino propionic acid-N-palmityl-N-oleyl-amide trihydrochloride, as a first helper lipid 1,2-diphytanoyl-sn-glycero-3-phosphoethanolamine and as a second helper lipid 1,2-distearoyl-sn-glycero-3-phosphoethanolamine-PEG2000.

In an embodiment of the first and the second aspect of the present invention the content of the second helper lipid is from about 0,05 mol% to 4,9 mol%, preferably about 1 to 2 mol% of the overall lipid content of the composition or of the lipoplex.

In an embodiment of any of the first and the second aspect of the present invention the composition contains from about 1 to 10 mol%, more preferably 1 to 7.5 mol% and most preferably 1 to 5 mol% of the conjugate of PEG and the ceramide of the overall lipid content of the composition or of the lipoplex.

In a preferred embodiment of the first and the second aspect of the present invention the ceramid is C8m and PEG is PEG 2000 and wherein the content of the conjugate of PEG and the ceramide is from about 1 to 5 mol% of the overall lipid content of the composition or of the lipoplex.

In an alternative preferred embodiment of the first and the second aspect of the present invention the ceramide is C8m and PEG is PEG5000 and wherein the content of the conjugate of PEG and ceramide is from about 1 to 5 mol% of the overall lipid content of the composition or of the lipoplex.

In an embodiment of the first and the second aspect of the present invention the content of the first lipid component is from about 42.5 mol% to 50 mol%, the content of the first helper lipid is from about 42.5 to 50 mol%, whereby the sum of the content of the first lipid component, of the first helper lipid and of the conjugate of PEG and ceramide is 100 mol%.

In an embodiment of any of the first and the second aspect of the present invention the composition further comprises a nucleic acid, preferably a functional nucleic acid which is more preferably a double-stranded ribonucleic acid and most preferably a nucleic acid selected from the group comprising RNAi, siRNA, siNA, antisense nucleic acid and ribozyme, whereby preferably the molar ration of RNAi to cationic lipid is from about 0 to 0.075, preferably from about 0.02 to 0.05 and even more preferably 0.037.

In an embodiment of any of the first and the second aspect of the present invention the carrier is an aqueous medium, preferably a sugar containing isotonic aqueous solution, and whereby the lipid composition contained in the carrier is present as a dispersion, preferably as a dispersion of liposomes and/or lipoplexes.

In an embodiment of any of the first and the second aspect of the present invention the lipid composition comprises about 50 mol% β-arginyl-2,3-diaminopropionic acid-N-palmityl-N-oleyl-amide trihydrochloride, about 48 to 49 mol% 1,2-diphytanoyl-sn-glycero-3-phosphoethanolamine, and about 1 to 2 mol% 1,2-distearoyl-sn-glycero-3-phosphoethanolamine-polyethylen-glycole and the carrier is an aqueous solution.

The problem underlying the present invention is solved in a third aspect by a lipoplex, which is preferably a composition according to any of the first and the second aspect of the present invention, or contained in such composition, comprising:
a) a positively charged liposome consisting of
   aa) about 50 mol% β-arginyl-2,3-diaminopropionic acid-N-palmityl-N-oleyl-amide trihydrochloride, preferably (β-(L-arginyl)-2,3-L-diaminopropionic acid-*N*-palmityl-*N*-oleyl-amide tri-hydrochloride)
   ab) about 48 to 49 mol% 1,2-diphytanoyl-sn-glycero-3-phosphoethanolamine (DPhyPE)
   ac) about 1 to 2 mol% 1,2-distearoyl-sn-glycero-3-phosphoethanolamine-polyethylen-glycole, preferably N-(Carbonyl-methoxypolyethyleneglycol-2000)-1,2-distearoyl-sn-glycero-3-phosphoethanolamine sodium salt; and
b) a functional nucleic acid, preferably a siRNA.

In an embodiment of the third aspect of the present invention the zeta-potential of the lipoplex is about 40 to 55 mV, preferably about 45 to 50 mV.

In an embodiment of the third aspect of the present invention the lipoplex has a size of about 80 to 200 nm, preferably of about 100 to 140 nm, and more preferably of about 110 nm to 130 nm, as determined by QELS.

In an alternative embodiment, the lipoplex has a size of about 60 nm, as preferably determined by QELS.

In an embodiment of the first and the second aspect of the present invention the composition comprises a lipoplex according to the third aspect of the present invention.

The problem underlying the present invention is solved in a fourth aspect by the use of a composition according to any of the first and the second aspect of the present invention or of a lipoplex according to the third aspect of the present invention, for the manufacture of a medicament.

In an embodiment of the fourth aspect of the present invention the medicament is for delivery of a nucleic acid, preferably a functional nucleic acid, to endothelium, preferably vascular endothelium.

In an embodiment of the fourth aspect of the present invention the medicament is for the treatment of an angiogenesis-dependent disease.

In an embodiment of the fourth aspect of the present invention the disease is a cancer disease, more preferably a solid tumor.

In an embodiment of the fourth aspect of the present invention the disease is selected from the group comprising neoplastic diseases, more preferably the diseases are selected from the group comprising bone cancer, breast cancer, prostate cancer, cancer of the digestive system, colorectal cancer, liver cancer, lung cancer, kidney cancer, urogenital cancer, pancreatic cancer, pituitary cancer, testicular cancer, orbital cancer, head and neck cancer, cancer of the central nervous system and cancer of the respiratory system.

In an embodiment of the fourth aspect of the present invention the medicament is used in combination with one or several other therapies.

In an embodiment of the fourth aspect of the present invention the therapy is selected from the group comprising chemotherapy, cryotherapy, hyperthermia, antibody therapy and radiation therapy.

The problem underlying the present invention is solved in a fifth aspect by the use of a composition according to any of the first and the second aspect or of a lipoplex according to the third aspect of the present invention, as a transferring agent.

In an embodiment of the fifth aspect of the present invention the transferring agent transfers a nucleic acid, whereby the nucleic acid is preferably the nucleic acid contained in the composition or the nucleic acid is preferably the nucleic acid component of the lipoplex.

In an embodiment of the fifth aspect of the present invention the nucleic acid is a functional nucleic acid, preferably a siRNA.

In an embodiment of the fifth aspect of the present invention the transferring agent is specific for endothelium, preferably vascular endothelium.

In an embodiment of the fifth aspect of the present invention the transferring agent transfers a pharmaceutically active component and/or a further constituent into a cell, preferably a mammalian cell and more preferably a human cell.

The problem underlying the present invention is solved in a sixth aspect by a method for transferring a pharmaceutically active compound and/or a further constituent into a cell or across a membrane, preferably a cell membrane, comprising the following steps:
- providing the cell or the membrane;
- providing a composition according to any of the first and the second aspect of the present invention or a lipoplex according to the third aspect of the present invention, whereby the composition optionally comprises the pharmaceutically active compound and/or the further constituent; and
- contacting the cell or the membrane with the composition according to any of the first and the second aspect of the present invention or a lipoplex according to the third aspect of the present invention.

In an embodiment of the sixth aspect of the present invention the method comprises as further step:
- detecting the pharmaceutically active compound and/or the further constituent in the cell and/or beyond the membrane.

The present inventors have surprisingly found that in contrast to naked or non-formulated siRNA molecules, siRNA-lipoplexes were strongly taken up by the vascular endothelium. The observed siRNA-lipoplex uptake in vivo was correlated with RNAi efficacy by means of knockdown analysis. A repeated systemic i.v. administration of target-specific siRNA-lipoplexes resulted in a clear knockdown of mRNA as well as protein of endothelium-specifically expressed target genes demonstrating the functional uptake and RNAi-mediated silencing activity. Therefore, the lipid composition described herein is particularly useful for specific delivery or targeting of the endothelium/ endothelial cells, more specifically vascular endothelium.

Furthermore, the present inventors have realized that with regard to the first lipid component a smaller amount of lipid compared to lipid compositions of the prior art is required to complex the siRNA molecules because of the lipid composition of the present invention. Also surprisingly using the lipid composition of the present invention, neither an unspecific interferon response, indicated by an increase in the expression of the interferon responsive OAS-1 and OAS-2 genes in HeLa cells, nor any induction of cytokines IFN-gamma and IL-12 has been observed using the lipid composition in accordance with the present invention.

Finally, the present inventors have surprisingly found that using the lipid composition of the present invention, there is not only a comparatively specific uptake of such composition and lipoplexes formed therefrom, but, more importantly, a cell-specific functional delivery and release from the endosome after endocytotic uptake by the endothelial cells. The latter is crucial insofar as only upon release of the functionally active agents such as a functional nucleic acid and in particular a siRNA, such functionally active agent can exert its effects at the cellular level.

In view of this, the lipid composition and lipoplex as disclosed herein, particularly if used as a pharmaceutical formulation for the delivery of a functional nucleic acid, including, but not limited to, siRNA can be used for the treatment and prevention, respectively, of various diseases, including, but not limited to, leukaemia and neo-angeogenesis dependent solid cancers, including but not limited to the carcinoma, sarcoma and lymphoma categories and other neoplasms such as any cancer and tumors, respectively, of, e.g., bone, breast, prostate, digestive system, colorectal, liver, lung, kideney, urogenital, pancreatic, pituitary, testicular, orbital, head and neck, central nervous system, respiratory. Further diseases for which such medicament and pharmaceutical formulation can be used are stroke, ulcers, atherosclerosis and rheumatoid arthritis, all of which are characterized by an either excessive or insufficient angiogenesis. In other words, the lipid composition and lipoplex, respectively, of the present invention can be used for both pro- and anti-angiogenic therapies and thus for the manufacture of a medicament for improving or increasing angiogenesis, or a medicament for decreasing angiogenesis.

Furthermore, this kind of medicament and pharmaceutical formulation, respectively, could be used in combination with other treatments, such as chemotherapy, cryotherapy, hyperthermia, antibody therapy such as monoclonal antibody therapy and VEGF-monoclonal antibody therapy in particular, radiation therapy, and the like.

Further specific diseases which, in principle, can be treated using such kind of pharmaceutical formulation and the medicament comprising such lipid composition and lipoplex, respectively, may be taken from the following list: Acute Lymphoblastic Leukemia (Adult), Acute Lymphoblastic Leukemia (Childhood), Acute Myeloid Leukemia (Adult) Acute Myeloid Leukemia (Childhood), Adrenocortical Carcinoma , Adrenocortical Carcinoma (Childhood), AIDS-Related Cancers, AIDS-Related Lymphoma, Anal Cancer, Astrocytoma (Childhood), Cerebellar Astrocytoma (Childhood) Cerebral, Bile Duct Cancer, Extrahepatic, Bladder Cancer, Bladder Cancer (Childhood), Bone Cancer, Osteosarcoma/Malignant Fibrous Histiocytoma, Brain Stem Glioma (Childhood) Brain Tumor (Adult), Brain Tumor, Brain Stem Glioma (Childhood), Brain Tumor, Cerebellar Astrocytoma (Childhood), Brain Tumor, Cerebral Astrocytoma/Malignant Glioma (Childhood), Brain Tumor, Ependymoma (Childhood), Brain Tumor, Medulloblastoma (Childhood), Brain Tumor, Supratentorial Primitive Neuroectodermal Tumors (Childhood), Brain Tumor, Visual Pathway and Hypothalamic Glioma (Childhood), Brain Tumor (Childhood), Breast Cancer , Breast Cancer (Childhood), Breast Cancer, Male, Bronchial Adenomas/Carcinoids (Childhood), Burkitt's Lymphoma,, Carcinoid Tumor (Childhood), Carcinoid Tumor,Gastrointestinal, Carcinoma of Unknown Primary, Central Nervous System Lymphoma, Primary, Cerebellar Astrocytoma (Childhood), Cerebral Astrocytoma/Malignant Glioma (Childhood), Cervical Cancer, Chronic Lymphocytic Leukemia, Chronic Myelogenous Leukemia, Chronic Myeloproliferative Disorders, Colon Cancer, Colorectal Cancer (Childhood), Cutaneous T-Cell Lymphoma, Endometrial Cancer, Ependymoma (Childhood), Esophageal Cancer, Esophageal Cancer (Childhood), Ewing's Family of Tumors, Extracranial Germ Cell Tumor (Childhood), Extragonadal Germ Cell Tumor, Extrahepatic Bile Duct Cancer, Eye Cancer, Intraocular Melanoma, Eye Cancer, Retinoblastoma, Gallbladder Cancer, Gastric (Stomach) Cancer, Gastric (Stomach) Cancer (Childhood), Gastrointestinal Carcinoid Tumor, Germ Cell Tumor, Extracranial, (Childhood), Germ Cell Tumor, Extragonadal, Germ Cell Tumor, Ovarian, Gestational Trophoblastic Tumor, Glioma (Adult), Glioma (Childhood) Brain Stem, Glioma (Childhood) Cerebral Astrocytoma, Glioma (Childhood) Visual Pathway and Hypothalamic, Hairy Cell Leukemia, Head and Neck Cancer, Hepatocellular (Liver) Cancer (Adult) (Primary),Hepatocellular (Liver) Cancer (Childhood) (Primary), Hodgkin's Lymphoma (Adult), Hodgkin's Lymphoma (Childhood), Hypopharyngeal Cancer, Hypothalamic and Visual Pathway Glioma (Childhood), Intraocular Melanoma, Islet Cell Carcinoma (Endocrine Pancreas), Kaposi's Sarcoma, Kidney (Renal Cell) Cancer, Kidney Cancer (Childhood), Laryngeal Cancer, Laryngeal Cancer (Childhood), Leukemia, Acute Lymphoblastic, (Adult), Leukemia, Acute Lymphoblastic (Childhood), Leukemia, Acute Myeloid (Adult), Leukemia, Acute Myeloid (Childhood), Leukemia, Chronic Lymphocytic Leukemia, Chronic Myelogenous, Leukemia, Hairy Cell, Lip and Oral Cavity Cancer, Liver Cancer (Adult) (Primary), Liver Cancer (Childhood) (Primary), Lung Cancer, Non-Small Cell, Lung Cancer, Small Cell, Lymphoma, AIDS-Related, Lymphoma, Burkitt's, Lymphoma, Cutaneous T-Cell, Lymphoma, Hodgkin's (Adult), Lymphoma, Hodgkin's (Childhood), Lymphoma, Non-Hodgkin's (Adult), Lymphoma, Non-Hodgkin's (Childhood), Lymphoma, Primary Central Nervous System, Macroglobulinemia, Waldenstrom's Malignant Fibrous Histiocytoma of Bone/Osteosarcoma, Medulloblastoma (Childhood), Melanoma, Melanoma, Intraocular (Eye), Merkel Cell Carcinoma, Mesothelioma (Adult) Malignant, Mesothelioma (Childhood), Metastatic Squamous Neck Cancer with Occult Primary, Multiple Endocrine Neoplasia Syndrome (Childhood), Multiple Myeloma/Plasma Cell Neoplasm, Mycosis Fungoides, Myelodysplastic Syndromes, Myelodysplastic/Myeloproliferative Diseases, Myelogenous Leukemia, Chronic, Myeloid Leukemia (Adult) Acute, Myeloid Leukemia (Childhood) Acute, Myeloma, Multiple, Myeloproliferative Disorders, Chronic, Nasal Cavity and Paranasal Sinus Cancer, Nasopharyngeal Cancer, Nasopharyngeal Cancer (Childhood), Neuroblastoma, Non-Hodgkin's Lymphoma (Adult), Non-Hodgkin's Lymphoma (Childhood), Non-Small Cell Lung Cancer, Oral Cancer (Childhood), Oral Cavity Cancer, Lip and Oropharyngeal Cancer, Osteosarcoma/Malignant Fibrous Histiocytoma of Bone, Ovarian Cancer, (Childhood), Ovarian Epithelial Cancer, Ovarian Germ Cell Tumor, Ovarian Low Malignant Potential Tumor, Pancreatic Cancer, Pancreatic Cancer (Childhood), Pancreatic Cancer, Islet Cell, Paranasal Sinus and Nasal Cavity Cancer, Parathyroid Cancer, Penile Cancer, Pheochromocytoma, Pineoblastoma and Supratentorial Primitive Neuroectodermal Tumors (Childhood), Pituitary Tumor, Plasma Cell Neoplasm/Multiple Myeloma, Pleuropulmonary Blastoma, Pregnancy and Breast Cancer, Pregnancy and Hodgkin's Lymphoma, Pregnancy and Non-Hodgkin's Lymphoma, Primary Central Nervous System Lymphoma, Prostate Cancer, Rectal Cancer, Renal Cell (Kidney) Cancer, Renal Cell (Kidney) Cancer (Childhood), Renal Pelvis and Ureter, Transitional Cell Cancer, Retinoblastoma, Rhabdomyosarcoma, (Childhood), Salivary Gland Cancer, Salivary Gland Cancer, (Childhood), Sarcoma, Ewing's, Sarcoma, Kaposi's, Sarcoma, Soft Tissue (Adult), Sarcoma, Soft Tissue (Childhood), Sarcoma, Uterine, Sezary Syndrome, Skin Cancer (non-Melanoma), Skin Cancer (Childhood), Skin Cancer (Melanoma), Skin Carcinoma, Merkel Cell, Small Cell Lung Cancer, Small Intestine Cancer, Soft Tissue Sarcoma, (Adult), Soft Tissue Sarcoma, (Childhood), Squamous Cell Carcinoma, Squamous Neck Cancer with Occult Primary, Metastatic Stomach (Gastric) Cancer, Stomach (Gastric) Cancer (Childhood), Supratentorial Primitive Neuroectodermal Tumors (Childhood), T-Cell Lymphoma, Cutaneous, Testicular Cancer, Thymoma (Childhood), Thymoma and Thymic Carcinoma, Thyroid Cancer Thyroid Cancer (Childhood), Transitional Cell Cancer of the Renal Pelvis and Ureter, Trophoblastic Tumor, Gestational, Ureter and Renal Pelvis, Transitional Cell Cancer, Urethral Cancer, Uterine Cancer, Endometrial, Uterine Sarcoma, Vaginal Cancer, Visual Pathway and Hypothalamic Glioma, (Childhood), Vulvar Cancer, Waldenström's Macroglobulinemia, Wilms' Tumor.

The lipid composition and lipoplex exhibiting this kind of specificity are characterized in the following.

The present inventors have surprisingly found that a very effective delivery in *in vivo* applications of nucleic acids, in particular small nucleic acids such as RNAi, siRNA and siNA, can be achieved by using a lipid composition comprising at least a first lipid component, at least a first helper lipid and a shielding compound. Preferably, the first helper lipid is a neutral, i. e. charge neutral or charge-free helper lipid. Such lipid composition is typically contained in a carrier, however, can also be present in a lyophilised form. The lipid composition contained in a carrier usually forms a dispersion. More preferably, the carrier is an aqueous medium or aqueous solution as also further characterised herein. The lipid composition typically forms a liposome in the carrier, whereby such liposome preferably also contains the carrier inside.

The lipid composition contained in the carrier and the carrier, respectively, preferably has an osmolarity of about 50 to 600 mosmole/kg, preferably about 250 - 350 mosmole/kg, and more preferably about 280 to 320 mosmole/kg.

The liposomes preferably formed by the first lipid component and optionally also by the first helper lipid, preferably in combination with the first lipid component, preferably exhibit a particle size of about 20 to 200 nm, preferably about 30 to 100 nm, and more preferably about 40 to 80 nm.

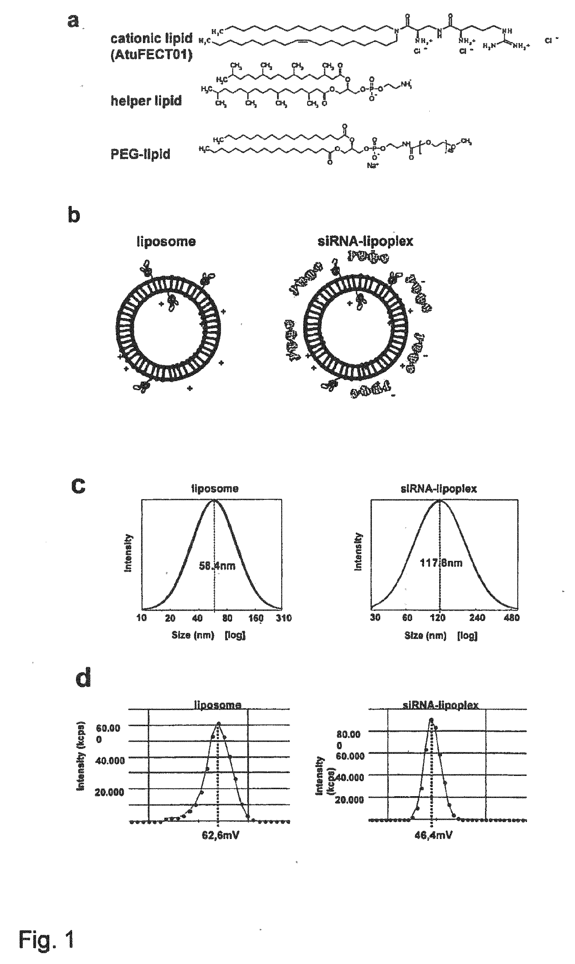

Furthermore, it will be acknowledged that the size of the particle follows a certain distribution. An insofar representative particle size distribution is depicted in Fig. 1c which as such is, in principle, also applicable to other mean sizes of particles, i. e. liposomes and lipoplexes, respectively.

A further optional feature of the lipid composition in accordance with the present invention is that the pH of the carrier is preferably from about 4.0 to 6.0. However, also other pH ranges such as from 4.5 to 8.0, preferably from about 5.5 to 7.5 and more preferably about 6.0 to 7.0 are within the present invention.

For realizing these particular features various measures may be taken as, as such, known to the person skilled in the art. For adjusting the osmolarity, for example, a sugar or a combination of sugars is particularly useful. Insofar, the lipid composition of the present invention may comprise one or several of the following sugars: sucrose, trehalose, glucose, galactose, mannose, maltose, lactulose, inulin and raffinose, whereby sucrose, trehalose, inulin and raffinose are particularly preferred. In a particularly preferred embodiment the osmolarity mostly adjusted by the addition of sugar is about 300 mosmole/kg which corresponds to a sucrose solution of 270 mM or a glucose solution of 280 mM. Preferably the carrier is isotonic to the body fluid into which such lipid composition is to be administered. As used herein the term that the osmolarity is mostly adjusted by the addition of sugar means that at least about 80 %, preferably at least about 90 % of the osmolarity is provided by said sugar or a combination of said sugars.

If the pH of the lipid composition of the present invention is adjusted, this is done by using buffer substances which, as such, are basically known to the one skilled in the art. Preferably, basic substances are used which are suitable to compensate for the basic characteristics of the cationic lipids and more specifically of the ammonium group of the cationic head group. When adding basic substances such as basic amino acids and weak bases, respectively, the above osmolarity is to be taken into consideration. The particle size of such lipid composition and the liposomes formed by such lipid composition is preferably determined by dynamic light scattering (QELS) such as, e. g., by using an N5 submicron particle size analyzer from Beckman Coulter according to the manufacturer's recommendation.

If the lipid composition contains one or several nucleic acid(s), such lipid composition usually forms a lipoplex complex, i. e. a complex consisting of a liposome and a nucleic acid. The more preferred concentration of the overall lipid content in the lipoplex in preferably isotonic 270 mM sucrose or 280 mM glucose is from about 0,01 to 100 mg/ml, preferably 0.01 to 40 mg/ml and more preferably 0.01 to 25 mg/ml. It is to be acknowledged that this concentration can be increased so as to prepare a reasonable stock, typically such increase is by a factor of 2 to 3. It is also within the present invention that based on this, a dilution is prepared, whereby such dilution is typically made such that the osmolarity is within the range specified above. More preferably, the dilution is prepared in a carrier which is identical or in terms of function and more specifically in terms of osmolarity similar to the carrier used in connection with the lipid composition or in which the lipid composition is contained. In the embodiment of the lipid composition of the present invention whereby the lipid composition also comprises a nucleic acid, preferably a functional nucleic acid such as, but not limited to, a siRNA, the concentration of the siRNA in the lipid composition is about 0.2 to 0.4 mg/ml, preferably 0.28 mg/ml, and the total lipid concentration is about 1.5 to 2.7 mg/ml, preferably 2.17 mg/ml. It is to be acknowledged that this mass ratio between the nucleic acid fraction and the lipid fraction is particularly preferred, also with regard to the charge ratio thus realized. In connection with any further concentration or dilution of the lipid composition of the present invention, it is preferred that the mass ratio and the charge ratio, respectively, realized in this particular embodiment is preferably maintained despite such concentration or dilution.

Such concentration as used, for example, in a pharmaceutical composition, can be either obtained by dispersing the lipid in a suitable amount of medium, preferably a physiologically acceptable buffer or any carrier described herein, or can be concentrated by appropriate means. Such appropriate means are, for example, ultra filtration methods including cross-flow ultrafiltration. The filter membrane may exhibit a pore width of 1.000 to 300.000 Da molecular weight cut-off (MWCO) or 5 nm to 1 µm. Particularly preferred is a pore width of about 10.000 to 100.000 Da MWCO. It will also be acknowledged by the one skilled in the art that the lipid composition more specifically the lipoplexes in accordance with the present invention may be present in a lyophilized form. Such lyophilized form is typically suitable to increase the shelve life of a lipoplex. The sugar added, among others, to provide for the appropriate osmolarity, is used in connection therewith as a cryo-protectant. In connection therewith it is to be acknowledged that the aforementioned characteristics of osmolarity, pH as well as lipoplex concentration refers to the dissolved, suspended or dispersed form of the lipid composition in a carrier, whereby such carrier is in principle any carrier described herein and typically an aqueous carrier such as water or a physiologically acceptable buffer, preferably an isotonic buffer or isotonic solution.

The shielding compound provides for a longer circulation time in vivo and thus allows for a better biodistribution of the nucleic acid containing lipid composition. By this mechanism, it is possible to target sites within a human or animal body, more specifically a mammalian body, which are comparatively remote from the site of administration of such lipid composition as the lipid composition is not immediately absorbed by the tissue surrounding the injection site, which is usually the endothelial lining of the vasculature when an intravenous administration is performed. The shielding compound as used herein is preferably a compound which avoids the immediate interaction of the lipid composition with serum compounds or compounds of other bodily fluids or cytoplasma membranes, preferably cytoplasma membranes of the endothelial lining of the vasculature into which the lipid composition is preferably administered. The term shielding also means that elements of the immune system or other defence or removal mechanisms of the body into which such lipid composition is administered, do not immediately interact with the lipid composition again increasing its circulation time in a living organism. Insofar, the shielding compound acts as an anti-opsinizing compound. Without whishing to be bound by any mechanism or theory, it seems that the shielding compound forms a cover or coat which reduces the surface area of the lipid composition available for interaction with its environment which would otherwise result in the lipid composition to fuse with other lipids or being bound by factors of the human and animal body, respectively, at a time which is too early for such interaction although it has to be acknowledged that at a later stage, i. e. after a prolonged time upon administration of the lipid composition, such interaction is usually preferred or desired at least to a certain extent so as to provide the delivery of the payload of the liposomes and lipoplexes, respectively. Another mechanism on which the observed efficacy of the shielding compound seems to be based is the shielding of the overall charge of the lipid composition and in particular of the lipid composition containing a nucleic acid, preferably a functional nucleic acid as defined herein and more preferably a siRNA, siNA and RNAi. Again, the interaction of the lipid composition seems to be affected, whereby the effect arises from the shielding of the charges rather than the shielding of the lipid components. In connection with the present disclosure, it is to be acknowledged that a composition can comprise as little as one lipid.

The shielding compound is preferably a biologically inert compound. More preferably, the shielding compound does not carry any charge on its surface or on the molecule as such. Particularly preferred shielding compounds are thus polyethylenglycoles, hydroxyethylglucose based polymers, polyhydroxyethyl starch (polyHES) and polypropylene, whereby any of said compounds preferably has a molecule weight from about 500 to 10000 Da, more preferably from about 2000 to 5000 Da.

It is an important feature of the present invention that the shielding compound is preferably removable from the lipid composition under in vivo conditions. Such removal can, in one embodiment, comprise the removal of the shielding compound per se, but may also comprise in a different embodiment the removal of the shielding compound together with the compound to which the shielding compound is preferably covalently linked. Such removal exposes the other components of the lipid composition or part thereof to the environment such as the animal and human body, and ultimately allows the release and delivery, respectively, of a compound such as a nucleic acid contained in the lipid composition. The term in vivo conditions preferably means the conditions existing in an animal, more preferably a mammalian body, or human body, preferably in any bodily fluid such as blood, liquor, interstitium and intracellular liquids, and/or those existing in an endosome and/or lysosome. Depending on the design of the shielding compound, the removal can be affected in various ways as will be described in more detail in the following.

A further effect arising from the loss of the shielding agent, particularly if the shielding agent is PEG, is due to the fact that providing lipid formulations with PEG which is also referred to as PEGylation, often results in an impaired functional delivery of the nucleic acid molecules to be delivered by such lipid formulation into the cytoplasm. The bulky presence of the PEG at the lipid formulation impairs the endosomal uptake of the liposomes typically formed by the lipid formulation or interferes with the necessary endosomal escape of the nucleic acid contained or associated with the lipid composition.

In one embodiment, the shielding compound is selected from the group comprising PEG, HEG, polyhydroxyethyl starch (polyHES) and polypropylene, which is attached, preferably covalently attached, to a ceramide. The ceramide interacts with the lipid compound and, if present, with a helper lipid. Insofar, the ceramide can be either the first helper lipid or a second helper lipid. It is preferred that the ceramide conjugated to PEG is different from the first helper lipid. The ceramide is typically embedded into the lipid fraction of the lipid composition preferably formed by the first lipid component and the first helper lipid and will be released due to the comparatively short hydrocarbon chain at a certain rate. When the ceramide is thus released from the lipid composition, so is the shielding agent. Therefore, in this embodiment, the in vitro conditions allow for the disintegration of the lipid formulation and thus provides a prolonged lifetime or circulation time in vivo of the lipid composition.

In a further embodiment, the shielding agent is attached to a nucleic acid contained in the lipid composition or associated therewith. Preferably, the shielding agent is covalently linked to the nucleic acid, most preferably through a linker. In a more preferred embodiment, the linker is designed such that it is cleaved under physiological conditions, i. e. conditions prevailing in an animal or human organism. In a preferred embodiment only a certain percentage of the nucleic acid is actually provided with such polymer through a linker. Without wishing to be bound by any theory, it seems that it is sufficient that only a certain number of nucleic acid molecules forming part of the lipid composition has to have such a bulky moiety so as to provide the shielding effect to said lipid composition. Preferably, the portion of nucleic acid molecules ranges from about 0 to 20 %, more preferably 3 to 10 %, and even more preferably from 6 to 10 %. Preferred contents of nucleic acids having the shielding agent (which is also referred to herein as the shielding compound) are from about 0 to 3 %, 3 to 6 %, 6 to 10 % and 10 to 20 %, whereby % as used in this paragraph and throughout the present application, if not indicated to the contrary, is mole %.

Such linker can be a single-stranded RNA linker, more preferably comprising 1 to 20 nucleotides which will be cleaved by RNA endonuclease activity existing in or under in vivo conditions. In a further embodiment, the linker is formed by a single-stranded DNA linker which is cleaved by DNA endonucleases also present in or under in vivo conditions. In further embodiments, the linker can be formed by a double-stranded RNA or a double-stranded DNA The stability of the linker consisting of a nucleic acid is typically as follows: ssRNA< dsRNA,< ssDNA< <dsDNA. Such stability variety allows for a specific design of the residual time of the nucleic acid thus modified and the lipid composition, respectively, comprising such linker.

In a further embodiment, the linker can be formed by an oligopeptide, polypeptide or protein which is cleaved by proteases present in or under in vivo conditions. A still further embodiment provides a linker comprising an S-S-linkage which is sensitive to redox conditions.

In an even still further embodiment the linker is a pH sensitive linker. The concept of a pH sensitive linker resides in the observation that upon internalisation of a lipid composition which can, in principle, be either a lipoplex or a liposome, any shielding agent which is clearly advantageous when it comes to the protection of the lipid composition prior to internalisation can impose a limitation of the further use of the compounds thus transferred into the cell. As used herein, the shielding agent is coupled to the pH sensitive linker, which is or comprises in a preferred embodiment an anionic moiety. In an acidic environment such as an endosome, the charge of such anionic moiety is changed. Because of this, the interaction of the pH sensitive linker with the cationic lipid contained in the lipid formulation based on electrostatics is also changed, usually reduced which results in a more or less gradual release of the pH sensitive linker from the cationic lipid comprising lipid formulation. Consequently, the liposome or lipoplex becomes devoid of the shielding agent and can thus exert its impact on and/or in the cell. This kind of linker comprises both linkers which are as such known in the art and new linkers of this kind, i. e. having this kind of characteristics. Preferably, such linker is any linker which has a charge characteristic which allows the linker to react as described above. Representatives of such pH sensitive linkers comprise, but are not limited to, oligo(glutamic acid), oligophenolates, and diethylene triamine penta acetic acid. Both the coupling of such linker to the shielding agent and the incorporation of such linker into the shielding agent which is then commonly referred to as a moiety of the shielding agent, is known to the ones skilled in the art.

The compound used as the first lipid component in the lipid composition according to the present invention which is also referred to herein as the compound(s) according to the present invention can, as depicted in Fig. 1, be regarded as to comprise a lipophilic group formed by the R1-N-R2 moiety, a linker group formed by the C(O)-CH(NH₃⁺) (CH₂)ₙ-NH moiety and a head group formed by the R3 moiety. The present inventor has surprisingly found that this kind of compound exhibiting a positive charge at the linker group is particularly suitable to transfer biologically active compounds over a cell membrane and preferably into cells, more preferably animal cells. Also, the present inventor has surprisingly found that the transfer mediated by the compounds according to the present invention will be particularly effective if the biologically active compound is a nucleic acid, more preferably siRNA and siNA.

As preferably used herein, the term alkyl refers to a saturated aliphatic radical containing from 8 to 20 carbon atoms, preferably 12 to 18 carbon atoms, or a mono- or polyunsaturated aliphatic hydrocarbon radical containing from 8 to 30 carbon atoms, containing at least one double and triple bond, respectively. Thus, in a preferred embodiment, the term alkyl also comprises alkenyl and alkinyl. Alky refers to both branched and unbranched, i. e. non-linear or straight chain alkyl groups. Preferred straight chain alkyl groups contain from 8 to 30 carbon atoms. More preferred straight chain alkyl groups contain from 12 to 18 carbon atoms. Preferred branched alkyl groups contain from 8 to 30 carbon atoms, whereby the number from 8 to 30 carbon atoms refers to the number of carbon atoms forming the backbone of such branched alkyl group. The backbone of the branched alkyl group contains at least one alkyl group as branching off from the backbone, with the alkyl group being defined as herein, more preferably with the alkyl group comprising short chain alkyl groups, more preferably comprising from 1 to 6, even more preferred 1 to 3 and most preferred 1 C atom. More preferred are branched alkyl groups containing 12 to 18 carbon atoms in the backbone with the branching alkyl groups being defined as in the foregoing. A particularly preferred alkyl group is the phytanyl group.

In an alternative embodiment, the alkyl is an unsaturated branched or unbranched alkyl group as defined above. More preferably, such unsaturated aliphatic hydrocarbon radical contains 1, 2 or 3 or 4 double bonds, whereby a radical having one double bond is particularly preferred. Most preferred is oleyl which is C18: 1delta9, i. e. an aliphatic hydrocarbon radical having 18 C atoms, whereby at position 9 a cis configured double bond is presented rather than a single bond linking C atom number 9 to C atom number 10.

As used herein, n is any integer between 1 and 4, which means that n may be 1, 2, 3 and 4. As used herein, m is any integer between 1 and 3, which means that m may be 1,2 and 3.

It is to be understood that the compounds according to the present invention are preferably cationic lipids. More preferably, any of the NH or NH₂ groups present in the compounds according to the present invention are present in a protonated form. Typically, any positive charge of the compound according to the present invention is compensated by the presence of an anion. Such anion can be a monovalent or polyvalent anion. Preferred anions are halides, acetate and trifluoroacetate. Halides as used herein are preferably fluorides, chlorides, iodides and bromides. Most preferred are chlorides. Upon association of the cationic lipid and the biologically active compound to be transferred into a cell, the halide anion is replaced by the biologically active compound which preferably exhibits one or several negative charges, although it has to be acknowledged that the overall charge of the biologically active compound is not necessarily negative.

It is to be acknowledged that any compound according to formula (I) comprises at least two asymmetric C atoms. It is within the present invention that any possible enantiomer of such compound is disclosed herein, i. e. in particular the R-R; S-S; R-S and S-R enantiomer.

The compounds according to the present invention can form a composition or be part of a composition, whereby such composition comprises a carrier. In such composition which is also referred to herein as lipid composition the compounds according to the present invention are also referred to as the lipid component(s). Such carrier is preferably a liquid carrier. Preferred liquid carriers are aqueous carriers and non-aqueous carriers. Preferred aqueous carriers are water, aqueous buffer systems, more preferably buffer systems having a physiological buffer strength and physiological salt concentration(s). Preferred non-aqueous carriers are solvents, preferably organic solvents such as ethanol, and tert.-butanol. Without wishing to be bound by any theory, any water miscible organic solvent can, in principle, be used. It is to be acknowledged that the composition, more particularly the lipid composition can thus be present as or form liposomes. It is within the present invention that the osmolarity and pH as specified in connection with the lipid composition of the present invention as well as the lipoplex concentration refers to a system comprising the lipid composition and lipoplex, respectively, and such carrier.

The composition according to the present invention may comprise one or more helper lipids which are also referred to herein as helper lipid components. The helper lipid components are preferably selected from the group comprising phospholipids and steroids. Phospholipids are preferably di- and monoester of the phosphoric acid. Preferred members of the phospholipids are phosphoglycerides and sphingolipids. Steroids, as used herein, are naturally occurring and synthetic compounds based on the partially hydrogenated cyclopenta[a]phenanthrene. Preferably, the steroids contain 21 to 30 C atoms. A particularly preferred steroid is cholesterol.

Particularly preferred helper lipids are 1,2-diphytanoyl-sn-glycero-3-phosphoethanolamine (DPhyPE) and 1,2-dioleoyl-sn-glycero-3-phosphoethanolamine (DOPE).

Particularly preferred compositions according to the present invention comprise any of β-arginyl-2,3-diaminopropionic acid-N-palmityl-N-oleyl-amide trihydrochloride [#6], β-arginyl-2,3-diaminopropionic acid-N-lauryl-N-myristyl-amide trihydrochloride [#11] or ε-arginyl-lysine-N-lauryl-N-myristyl-amide trihydrochloride [#15] in combination with DPhyPE, whereby the content of DPhyPE is from about 90 to 20 mol %, preferably 80 mol %, 65 mol %, 50 mol % and 35 mol %, whereby the term mol % refers to the percentage of the overall lipid content of the composition, i. e. the lipid content of the composition including the cationic lipid according to the present invention and any additional lipid, including, but not limited to, any helper lipid.

It is within the present invention that the composition according to the present invention preferably comprises the compound according to the present invention and/or one or several of the helper lipid(s) as disclosed herein, whereby either the compound according to the present invention, i. e. the cationic lipid, and/or the helper lipid component is present as a dispersion in an aqueous medium. Alternatively, the compound according to the present invention, i. e. the cationic lipid, and/or the helper lipid component is/are present as a solution in a water miscible solvent. As an aqueous medium, preferably any of the aqueous carrier as disclosed herein is used. Preferred water miscible solvents are any solvent which form a homogenous phase with water in any ratio. Preferred solvents are ethanol and tert.-butanol. It is to be acknowledged that the composition, more particularly the lipid composition can thus be present as or form liposomes.

It is to be acknowledged that the composition according to the present invention in its various embodiments is and may thus also be used as a pharmaceutical composition. In the latter case, the pharmaceutical composition comprises a pharmaceutically active compound and optionally a pharmaceutically acceptable carrier. Such pharmaceutically acceptable carrier may, preferably, be selected from the group of carriers as defined herein in connection with the composition according to the present invention. It will be understood by those skilled in the art that any composition as described herein may, in principle, be also used as a pharmaceutical composition provided that its ingredients and any combination thereof is pharmaceutically acceptable. A pharmaceutical composition comprises a pharmaceutically active compound. Such pharmaceutically active compound can be the same as the further constituent of the composition according to the present invention which is preferably any biologically active compound, more preferably any biologically active compound as disclosed herein. The further constituent, pharmaceutically active compound and/or biologically active compound are preferably selected from the group comprising peptides, proteins, oligonucleotides, polynucleotides and nucleic acids.

Preferably, any such biologically active compound is a negatively charged molecule. The term negatively charged molecule means to include molecules that have at least one negatively charged group that can ion-pair with the positively charged group of the cationic lipid according to the present invention, although the present inventor does not wish to be bound by any theory. In principle, the positive charge at the linker moiety could also have some effect on the overall structure of either the lipid as such or any complex formed between the cationic lipid and the negatively charged molecule, i. e. the biologically active compound. Apart from that, the additional positive charge introduced into the lipid according to the present invention compared to the cationic lipids disclosed in US patent 6,395,713, should contribute to an increased toxicity of this lipid as taught by Xu Y, Szoka FC Jr.; Biochemistry; 1996 May 07, 35 (18): 5616-23. In contrast to what the one skilled in the art would have expected from this document of the prior art the compounds according to the present invention are particularly suitable for the various purposes disclosed herein and are in particular devoid of any toxicity or any increased toxicity.

A peptide as preferably used herein is any polymer consisting of at least two amino acids which are covalently linked to each other, preferably through a peptide bond. More preferably, a peptide consists of two to ten amino acids. A particularly preferred embodiment of the peptide is an oligopeptide which even more preferably comprises from about 10 to about 100 amino acids. Proteins as preferably used herein are polymers consisting of a plurality of amino acids which are covalently linked to each other. Preferably such proteins comprise about at least 100 amino acids or amino acid residues.

A preferred protein which may be used in connection with the cationic lipid and the composition according to the present invention, is any antibody, preferably any monoclonal antibody.

Particularly preferred biologically active compounds, i. e. pharmaceutically active compounds and such further constituent as used in connection with the composition according to the present invention are nucleic acids. Such nucleic acids can be either DNA, RNA, PNA or any mixture thereof. More preferably, the nucleic acid is a functional nucleic acid. A functional nucleic acid as preferably used herein is a nucleic acid which is not a nucleic acid coding for a peptide and protein, respectively. Preferred functional nucleic acids are siRNA, siNA, RNAi, antisense-nucleic acids, ribozymes, aptamers and spiegelmers which are all known in the art.

siRNA are small interfering RNA as, for example, described in international patent application PCT/EP03/08666. These molecules typically consist of a double-stranded RNA structure which comprises between 15 to 25, preferably 18 to 23 nucleotide pairs which are base-pairing to each other, i. e. are essentially complementary to each other, typically mediated by Watson-Crick base-pairing. One strand of this double-stranded RNA molecule is essentially complementary to a target nucleic acid, preferably an mRNA, whereas the second strand of said double-stranded RNA molecule is essentially identical to a stretch of said target nucleic acid. The siRNA molecule may be flanked on each side and each stretch, respectively, by a number of additional oligonucleotides which, however, do not necessarily have to base-pair to each other.

RNAi has essentially the same design as siRNA, however, the molecules are significantly longer compared to siRNA. RNAi molecules typically comprise 50 or more nucleotides and base pairs, respectively.

A further class of functional nucleic acids which are active based on the same mode of action as siRNA and RNAi is siNA. siNA is, e. g., described in international patent application PCT/EP03/074654. More particularly, siNA corresponds to siRNA, whereby the siNA molecule does not comprise any ribonucleotides.

Antisense nucleic acids, as preferably used herein, are oligonucleotides which hybridise based on base complementarity with a target RNA, preferably mRNA, thereby activating RNaseH. RNaseH is activated by both phosphodiester and phosphothioate-coupled DNA. Phosphodiester-coupled DNA, however, is rapidly degraded by cellular nucleases with the exception of phosphothioate-coupled DNA. Antisense polynucleotides are thus effective only as DNA-RNA hybrid complexes. Preferred lengths of antisense nucleic acids range from 16 to 23 nucleotides. Examples for this kind of antisense oligonucleotides are described, among others, in US patent 5,849,902 and US patent 5,989,912.

A further group of functional nucleic acids are ribozymes which are catalytically active nucleic acids preferably consisting of RNA which basically comprise two moieties. The first moiety shows a catalytic activity, whereas the second moiety is responsible for a specific interaction with the target nucleic acid. Upon interaction between the target nucleic acid and the second moiety of the ribozyme, typically by hybridisation and Watson-Crick base-pairing of essentially complementary stretches of bases on the two hybridising strands, the catalytically active moiety may become active which means that it cleaves, either intramolecularly or intermolecularly, the target nucleic acid in case the catalytic activity of the ribozyme is a phosphodiesterase activity. Ribozymes, the use and design principles thereof are known to the ones skilled in the art and, for example, described in Doherty and Doudna (Annu. Ref. Biophys. Biomolstruct. 2000; 30: 457-75).

A still further group of functional nucleic acids are aptamers. Aptamers are D-nucleic acids which are either single-stranded or double-stranded and which specifically interact with a target molecule. The manufacture or selection of aptamers is, e. g., described in European patent EP 0 533 838. In contrast to RNAi, siRNA, siNA, antisense-nucleotides and ribozymes, aptamers do not degrade any target mRNA but interact specifically with the secondary and tertiary structure of a target compound such as a protein. Upon interaction with the target, the target typically shows a change in its biological activity. The length of aptamers typically ranges from as little as 15 to as much as 80 nucleotides, and preferably ranges from about 20 to about 50 nucleotides.

Another group of functional nucleic acids are spiegelmers as, for example, described in international patent application WO 98/08856. Spiegelmers are molecules similar to aptamers. However, spiegelmers consist either completely or mostly of L-nucleotides rather than D-nucleotides in contrast to aptamers. Otherwise, particularly with regard to possible lengths of spiegelmers, the same applies to spiegelmers as outlined in connection with aptamers.

As mentioned previously, the present inventor has surprisingly found that the compound according to the present invention and the respective compositions comprising such compound can be particularly effective in transferring RNAi, and more particularly siRNA and siNA into an endothelial cell. It is to be noted that although not wishing to be bound by any theory, due to the particular mol percentages of the helper lipid(s) contained in the lipid compositions according to the present invention, which helper lipid can be either a PEG-free helper lipid or in a particular embodiment a PEG-containing helper lipid, surprising effects can be realised, more particularly if the content of any of this kind of helper lipid is contained within the concentration range specified herein. In connection therewith, it is particularly noteworthy that if the composition according to the present invention contains a helper lipid comprising a PEG moiety, any delivery or transfection action using such PEG-derived helper lipid containing composition is particularly effective in delivering nucleic acid, particularly RNAi molecules, most particularly siRNA, siNA, antisense nucleotides and ribozymes.

The reason for this is that the present inventors have surprisingly found that liposomes formed by at least the first lipid component and at least a first helper lipid and which contain more than about 4% of PEG-containing helper lipid(s) are not active, whereas liposomes with less than 4% (preferably less than 3% but more than 0 %) do mediate functional delivery although a certain extent of delivery is, in principle, also observable beyond those limits. Basically, the present inventors have discovered that the specific amount of PEG in the lipid compositions according to the present invention is suitable to provide for an effective transfection of and delivery into, respectively, endothelial cells.

In a further aspect the present inventors have surprisingly found that the lipid compositions according to the present invention which are preferably present as lipoplexes or liposomes, preferably show an overall cationic charge and thus an excess of at least one positive charge. More preferably, the lipid compositions exhibit a charge ratio negative : positive of from about 1: 1.3 to 1:5. Therefore, the present invention is thus related in a further aspect to any lipid composition comprising at least one cationic lipid and a nucleic acid, preferably a RNAi, siRNA or siNA or any other of the functional nucleic acids defined herein, having a charge ratio negative : positive of from about 1: 1.3 to 1:5. The cationic lipid is preferably any cationic lipid described herein. The lipid composition comprises in a preferred embodiment any helper lipid or helper lipid combination as described herein.

The present inventors have also found that in particular the molar ratio of siRNA and the cationic lipid can be crucial for the successful application of the lipid composition according to the present invention, especially in view of what has been said above in relation to the cationic overall charge of the nucleic acid containing lipid formulations. Without wishing to be bound by any theory it seems that 1 mole of cationic lipid, particularly as disclosed herein, can provide for a maximum of three positive charges per molecule, whereas the nucleic acid and more particularly the siRNA molecules as disclosed herein, provide for a maximum of 40 negative charges per molecule. In order to reach an overall positive charge of the siRNA containing lipid formulations according to the present invention, the molar ratio can preferably range from 0 to a maximum of 0.075. A preferred molar ratio range is from about 0.02 to 0.05 and even more preferred is a molar ratio range of about 0.037. In a further embodiment of the composition and lipoplex, respectively, of the invention the ratio of the mass of the overall lipid to the mass of the siRNA is typically 2:1 to 1000:1 (m/m), whereby a ratio of 5:1 to 15:1 (m/m) is preferred and a ratio of 6:1 to 9:1 (m/m) is even more preferred.

It is within the present invention that the composition and more particularly the pharmaceutical composition may comprise one or more of the aforementioned biologically active compounds which may be contained in a composition according to the present invention as pharmaceutically active compound and as further constituent, respectively. It will be acknowledged by the ones skilled in the art that any of these compounds can, in principle, be used as a pharmaceutically active compound. Such pharmaceutically active compound is typically directed against a target molecule which is involved in the pathomechanism of a disease. Due to the general design principle and mode of action underlying the various biologically active compounds and thus the pharmaceutically active compounds as used in connection with any aspect of the present invention, virtually any target can be addressed. Accordingly, the compound according to the present invention and the respective compositions containing the same can be used for the treatment or prevention of any disease or diseased condition which can be addressed, prevented and/or treated using this kind of biologically active compounds. It is to be acknowledged that apart from these biologically active compounds also any other biologically active compound can be part of a composition according to any embodiment of the present invention. Preferably such other biologically active compound comprises at least one negative charge, preferably under conditions where such other biologically active compound is interacting or complexed with the compound according to the present invention, more preferably the compound according to the present invention which is present as a cationic lipid.

As used herein, a biologically active compound is preferably any compound which is biologically active, preferably exhibits any biological, chemical and/or physical effects on a biological system. Such biological system is preferably any biochemical reaction, any cell, preferably any animal cell, more preferably any vertebrate cell and most preferably any mammalian cell, including, but not limited to, any human cell, any tissue, any organ and any organism. Any such organism is preferably selected from the group comprising mice, rats, guinea pigs, rabbits, cats, dogs, sheep, pigs, goats, cows, horses, poultry, monkeys and humans.

It is also within the present invention that any of the compositions according to the present invention, more particularly any pharmaceutical composition according to the present invention may comprise any further pharmaceutically active compound(s).

The composition, particularly the pharmaceutical composition according to the present invention can be used for various forms of administration, whereby local administration and systemic administration are particularly preferred. Even more preferred is a route of administration which is selected from the group, comprising intramuscular, percutaneous, subcutaneous, intravenous and pulmonary administration. As preferably used herein, local administration means that the respective composition is administered in close spatial relationship to the cell, tissue and organ, respectively, to which the composition and the biologically active compound, respectively, is to be administered. As used herein, systemic administration means an administration which is different from a local administration and more preferably is the administration into a body fluid such as blood and liquor, respectively, whereby the body liquid transports the composition to the cell, tissue and organ, respectively, to which the composition and the biologically active compound, respectively, is to be delivered. However, an ex vivo administration such as in case of organ transplantation, is also comprised by the present invention.

As used herein, the cell across the cell membrane of which a biologically active compound is to be transferred by means of the compound and composition according to the present invention, respectively, is preferably an eukaryotic cell, more preferably a vertebrate cell and even more preferably a mammalian cell. Most preferably the cell is a human cell. In any case such cell is an endothelial cell.

Any medicament which can be manufactured using the compound and composition according to the present invention, respectively, is for the treatment and prevention of a disease in a patient. Preferably such patient is a vertebrate, more preferably a mammal and even more preferably such mammal is selected from the group comprising mice, rats, dogs, cats, guinea pigs, rabbits, sheep, pigs, goats, cows, horses, poultry monkeys and humans. In a further aspect the compound and composition according to the present invention can be used as a transferring agent, more preferably as a transfection agent.

As preferably used herein a transferring agent is any agent which is suitable to transfer a compound, more preferably a biologically active compound such as a pharmaceutically active compound across a membrane, preferably a cell membrane and more preferably transfer such compound into a cell as previously described herein. Even more preferably, such transfer also comprises the release from any endosome and/or lysosome. In a preferred embodiment the term cell membrane shall also comprise mebranes inside the cell such as vesicular membrane, membranes of the endosome and lysosome.

In a still further aspect the present invention is related to a method for transferring, more particularly transfecting, a cell with a biologically active compound. In a first step, whereby the sequence of steps is not necessarily limited and in particular not limited to the sequence of steps outlined in the following, the cell and the membrane and cell, respectively, is provided. In a second step, a compound or composition according to the present invention is provided as well as a biologically active compound such as a pharmaceutically active compound. This reaction can be contacted with the cell and the membrane, respectively, and due to the biophysical characteristics of the compound and the composition according to the present invention, the biologically active compound will be transferred from one side of the membrane to the other one, or in case the membrane forms a cell, from outside the cell to within the cell. It is within the present invention that prior to contacting the cell and the membrane, respectively, the biologically active compound and the compound or composition according to the present invention, are contacted, whereupon preferably a complex is formed and such complex is contacted with the cell and the membrane, respectively.

In a further aspect of the present invention the method for transferring a biologically active compound and a pharmaceutically active compound, respectively, comprises the steps of providing the cell and the membrane, respectively, providing a composition according to the present invention and contacting both the composition and the cell and the membrane, respectively. It is within the present invention that the composition may be formed prior or during the contacting with the cell and the membrane, respectively.

In an embodiment of any method for transferring a biologically active compound as disclosed herein, the method may comprise further steps, preferably the step of detecting whether the biologically active compound has been transferred. Such detection reaction strongly depends on the kind of biologically active compounds transferred according to the method and will be readily obvious for the ones skilled in the art. It is within the present invention that such method is performed on any cell, tissue, organ and organism as described herein.

It is to be acknowledged that in a further embodiment the shielding agent is attached, as described herein, to the lipid component of the lipid composition according to the present invention, preferably to the cationic lipid.

As used herein, the term treatment of a disease also comprises prevention of such disease.

The present invention is further illustrated by the following figures and examples from which further features, embodiments and advantages may be taken.
Fig. 1a shows the structures of the constituents of the lipid composition in accordance with the present invention.
Fig. 1b shows a schematic representation of a liposome formed by the lipid composition according to the present invention and the siRNA lipoplex according to the present invention formed by the lipid composition together siRNA molecules indicating that the siRNA molecules are forming a complex predominantly with the outer surface of the liposome rather than being contained in the liposome, whereby the negatively charged siRNAs are complexed by electrostatic interaction with the positive charges of the cationic lipid.
Fig. 1c shows a diagram depicting the size of a liposome formed by the lipid composition in accordance with the present invention and the siRNA lipoplex in accordance with the present invention.
Fig. 1d shows a diagram depicting the zeta potential of a liposome formed by the lipid composition in accordance with the present invention and the siRNA lipoplex in accordance with the present invention.
Fig. 2a shows the result of a Western blot analysis of concentration dependent inhibition of PKN3 protein expression with lipoplexed siRNAs and naked siRNA, respectively, in HeLa cells, whereby PTEN served as a loading control.
Fig. 2b shows pictures taken by confocal microscopy of HeLa cells treated with siRNAs labelled with Cy3 and administered either naked or as a lipoplex in accordance with the present invention.
Fig. 3a shows the result of a Western blot analysis using liposomal formulations containing different mol% of PEG.
Fig. 3b shows confocal microscopy pictures of cellular uptake of siRNA-Cy3-lipoplexes with 0, 1, 2 and 5 mol% PEG; EOMA cells were transfected with fluorescently labelled siRNA-lipoplexes; note: at 5 mol% PEG, most of the lipoplex decorates the surface of the cell (arrows).
Fig. 3c shows the result of a Western blot analysis and more specifically immunoblots with extracts from HUVEC cells transfected with different amounts of PEGylated (1mol %) and non-PEGylated siRNA^{PKN3}-lipoplex (upper panel) or siRNA^{PTEN}-lipoplex (lower panel); the final concentration of siRNA is indicated (1-20 nM; ut: untreated); immunoblots were probed with anti-PTEN and anti-PKN3.
Fig. 3d shows various diagrams indicating the body weight development of nude mice treated with different formulations over a five days period, whereby the mice were treated (single i.v. injections on day 1-5) with PEGylated (triangle) or non-PEGylated (squares) lipoplexes of siRNA^{Luc}, siRNA^{PKN3}, siRNA^{CD31} and siRNA^{PTEN} over a 5 days period; shown are the relative changes in body weight as mean ± s.e.m. from 7 mice per treatment group.
Fig. 3e shows a diagram indicating the result of an IL-12 ELISA of blood samples from C57/BL6 mice (2 mice per group) after single treatment with poly(I:C) or indicated siRNA-lipoplexes for 2 (dark grey) or 24 (light grey) hours.
Fig. 4a shows epifluorescence microscopy pictures of paraffin embedded sections visualizing the distribution of naked (middle row) or lipoplexed (lower row) siRNA-Cy3 (1.88 mg/kg) in different tissues 20 minutes after tail vein injection.
Fig. 4b shows pictures of epifluorescence microscopy of heart, lung, spleen and liver analyzed at different time points after single systemic i.v. administration of siRNA-Cy3-lipoplex; tissue samples were recorded at identical microscopy settings (for each organ); size bars: 100µm; in the case of liver and spleen: 200µm.
Fig. 4c shows confocal microscopy pictures of endothelial cell distribution in the heart as revealed by IHC using anti-CD31 antibody (left picture) decorating cross and longitudinal sections of capillaries (arrow); Cy3-fluorescence staining of endothelial cells in the vasculature (right picture, arrow).
Fig. 4d shows pictures of confocal microscopy illustrating endothelial cell distribution in the lung as revealed by IHC using anti-CD31 antibody (left picture) decorating the lung capillaries in the lung (arrow, vessel; double-arrow, alveolar macrophages); Cy3-fluorescence staining of endothelial cells in the vasculature (right picture, arrow;); alveolar macrophages also show strong fluorescence (right picture, small arrows).
Fig. 5a shows a diagram illustrating knockdown of mRNA levels for Tie2 (right diagram) or CD31 (left diagram) in lung, heart and liver tissue from animals treated daily on four consecutive days with sucrose, siRNA^{CD31}-, siRNA^{PTEN}- or siRNA^{Tie2}-lipoplex as quantified by TaqMan RT-PCR; the indicated mRNA levels are shown relative to the sucrose group and normalized to the mRNA level of CD34; the values are means ± s.e.m. of the ratios from all animals per group.
Fig. 5b shows immunoblots of lung, heart, liver protein extracts from 6-7 individual mice treated with either siRNA^{CD31}-lipoplex or siRNA^{Tie2}-lipoplex were probed with anti-Tie2, or anti-CD31 and anti-PTEN (loading control); note: levels in protein expression changes only in Tie-2 or CD31 according to the lipoplex applied, while no changes in PTEN expression levels occurred.
Fig. 5c shows a diagram illustrating s-Tie2 as a function of various formulations and more specifically the decrease of soluble Tie2 (s-Tie2) after four daily treatments with siRNA^{Tie2}-lipoplex (day 5, right diagram), but not with control lipoplexes (siRNA^{PTEN}, siRNA^{CD31}) and sucrose; the measured s-Tie2 levels of individual mice per treatment group are indicated as rhombuses; soluble Tie2 levels from mice before treatment (day 0) are shown in the left diagram, after treatment in the right diagram; the mean value of s-Tie2 for each treatment group is shown as lines.
Fig. 6 shows a diagram indicating the size distribution of liposomes having a mean particle size of about 85 nm suitable for the preparation of lipoplexes having a mean particle size of about 120 nm.
Fig. 7 shows a diagram indicating the size distribution of lipoplexes having a mean particle size of about 120 nm.
Fig. 8. shows a diagram indicating the size distribution of several batches of liposomes having a mean particle size of about 30 nm suitable for the preparation of lipoplexes having a mean particle size of about 60 nm.
Fig. 9 shows a diagram indicating the size distribution of lipoplexes having a mean particle size of about 60 nm.

### Example 1: Material and Methods

### siRNAs

The siRNA molecules (AtuRNAi) used in this study are blunt, 19-mer double-stranded RNA oligonucleotides stabilized by alternating 2'-O-methyl modifications on both strands (for details see (Czauderna et al., 2003)) and were synthesized by BioSpring (Frankfurt a. M., Germany). siRNA sequences used in this study are listed in Table 1.

**Table 1: siRNA sequences**

| **siRNA name** | **sequence 5' to 3'** |
|---|---|
| PKN3 s | gagagccuguacugcgaga |
| PKN3 as | ucucgcaguacaggcucuc |
| | |
| PTEN s | ccaccacagcuagaacuua |
| PTEN as | uaaguucuagcuguggugg |
| | |
| CD31 s | uuccguucuagaguaucug |
| CD31 as | cagauacucuagaacggaa |
| | |
| PTEN s | ccaccacagcuagaacuua |
| PTEN as-Cy3 | uaaguucuagcuguggugg-Cy3 |
| | |
| PTEN s | ccaccacagcuagaacuua |
| PTEN as-Cy5 | uaaguucuagcuguggugg-Cy5 |
| | |
| Luciferase s | cguacgcggaauacuucga |
| Luciferase as | ucgaaguauuccgcguacg |
| | |
| Tie2 s | auaucugggcaaaugaugg |
| Tie2 as | Ccaucauuugcccagauau |

| | |
|---|---|
| Nucleotides with 2'-O-methyl modifications are underlined. S stands for the sense strand which is also referred to herein as the first strand; and As stands for the antisense strand which is also referred to herein as the second strand. | |

### Preparation and characterization of siRNA-lipoplexes

Cationic liposomes comprising the novel cationic lipid AtuFECT01 which is β-*L*-arginyl-2,3-*L-*diaminopropionic acid-*N*-palmityl-*N*-oleyl-amide trihydrochloride, Atugen AG (Berlin), the neutral/helper lipid phospholipid 1,2-diphytanoyl-sn-glycero-3-phosphoethanolamine (DPhyPE) (Avanti Polar Lipids Inc., Alabaster, AL) and the PEGylated lipid N-(Carbonyl-methoxypolyethyleneglycol-2000)-1,2-distearoyl-sn-glycero-3-phospho-ethanolamine sodium salt (DSPE-PEG) (Lipoid GmbH, Ludwigshafen, Germany) in a molar ratio of 50/49/1 were prepared by lipid film re-hydration in 300mM sterile RNase-free sucrose solution to a total lipid concentration of 4.34 mg/ml 300mM sucrose, pH=4.5-6.0. Subsequently the multilamellar dispersion was further processed by high pressure homogenization (22 cycles at 750 bar and 5 cycles at 1000 bar) using an EmulsiFlex C3 device (Avestin, Inc., Ottawa, Canada). To generate siRNA-lipoplexes (AtuPLEX) the obtained liposomal dispersion was mixed with an equal volume of a 0.5625 mg/ml solution of siRNA in 300 mM sucrose, resulting in a calculated charge ratio of nucleic acid backbone phosphates to cationic lipid nitrogen atoms of approximately 1 to 4. The size of the liposome and the lipoplex-dispersion was determined by Quasi Elastic Light Scattering (N5 Submicron Particle Size Analyzer, Beckman Coulter, Inc., Miami, FL) and the zeta potential was measured using a Zetasizer Nano-ZS (Malvern Instruments, Worcestershire, UK).

For siRNA-lipoplex double labelling, the fluorescently labeled liposomes were generated by adding the fluorescently-labeled tracer lipid TexasRed^{®}-1,2-dihexadecanoyl-*sn*-glycero-3-phosphoethanolamine, triethylammonium salt (TexasRed^{®}-DHPE; Molecular Probes) at following ratio: 50 mol% cationic lipid (AtuFECT01)/44 mol% helper lipid DPhyPE/1 mol% DSPE-PEG/5 mol% TexasRed^{®}-DHPE. The liposomes were processed by 51 extrusion cycles through a 400 nm polycarbonate membrane prior mixing resulting in a final concentration of 2.17 mg/ml total for the lipids and 0.28 mg/ml siRNA (a 200µl injection into a 30g mouse represents a dose of 1.88 mg/kg siRNA and 14.5 mg/kg lipid).

### In vitro transfection and immunoblotting

Human, HeLa and murine EOMA cell lines were obtained from American Type Culture Collection and cultivated according to the ATCC's recommendation. Cell lines were transfected with siRNA using the cationic liposomes described above. Briefly, about 12 hours after cell seeding different amounts of siRNA-lipoplex solution diluted in 10% serum containing medium were added to the cells to achieve transfection concentrations in a range of 1-50 nM siRNA. After transfection (48 h) cells were lysed and subjected to immunoblotting as described (Klippel et al., 1998). For total protein extraction tissues were dissected and instantly snap-frozen in liquid nitrogen. 20 mg of tissue were homogenized in a Mixer Mill MM 301 (Retsch GmbH, Haan, Germany) using tungsten carbide beads (Qiagen) and proteins were extracted in NP40-lysis buffer. Protein concentration was determined with a DC Protein Assay (BioRad) and equal amounts were loaded for immunobot analysis using the following antibodies: Rabbit anti-PTEN (Ab-2, Neomarkers), monoclonal p110α (Klippel et al., 1994), rabbit anti-PKN3 (Leenders et al., 2004), goat anti-CD31 (Santa Cruz Biotechnology), rabbit anti Tie-2 (Cell Signaling Technology).

### siRNA-Cy3 uptake experiments in cell culture and mice

For uptake studies of non-formulated siRNA-Cy3 molecules in cell culture , cells were incubated with defined amounts of siRNA solution overnight in serum-containing medium. Uptake of lipoplexed siRNA-Cy3 was carried out by transfection overnight as mentioned above. Treated cells were rinsed with ice cold PBS and fixed in 4% formaldehyde/PBS solution for 15 minutes prior to microscopy. *In vivo* delivery experiment using fluorescently labeled siRNA-Cy3 was carried out by administering formulated and naked siRNA intravenously. Mice were treated with a single 200µl i.v. injection at a final dose of 1.88 mg/kg siRNA-Cy3 and 14.5 mg/kg lipid and were sacrificed at defined time-points and fluorescence uptake examined by microscopy on either formalin fixed, paraffin embedded or OCT mounted frozen tissue sections.

### Histological analysis and microscopy

After mice were sacrificed, tissues were instantly fixed in 4.5 % buffered formalin for 16 hours and consequently processed for paraffin embedding. 4 µm sections were cut and placed on glass slides. Tissue sections were stained with goat polyclonal anti-CD31/PECAM-1 (Santa Cruz Biotechnology) (alternatively for cryosections rat CD31, Pharmingen) to visualize endothelial cells in paraffin sections. Immunohistochemistry and hematoxylin/eosin (H+E) staining on paraffin tissue sections were performed according to standard protocols. For *in vivo* uptake studies of fluorescently labeled siRNAs, paraffin sections were directly examined by epifluorescence with a Zeiss Axioplan microscope. Images were recorded and processed using the Zeiss LSM5 imaging software. In depth microscopic analysis of siRNA uptake was performed with a Zeiss LSM510 Meta confocal microscope. For this, sections were deparaffinized with xylene, rehydrated through graded ethanol washes, counterstained with Sytox Green dye (Molecular Probes 100 nM), rinsed and finally mounted in FluorSave (Calbiochem) for microscopy.

### mRNA quantification by RT-PCR (TaqMan)

Tissues were dissected and instantly snap-frozen in liquid nitrogen. Approximately 20mg of tissue was homogenized in a Mixer Mill MM 301 (Retsch GmbH, Haan, Germany) using tungsten carbide beads (Qiagen) and total RNA was prepared with the Invisorb Spin Tissue RNA Mini Kit (Invitek, Berlin, Germany). Depending on the tissue 25 to 100ng total RNA was used for quantitative TaqMan RT-PCR with the following amplicon sets (BioTez GmBH, Berlin, Germany) (UPR: upper primer, LWR: lower primer, PRB: probe): CD31-specific for mouse mRNA, UPR 5'GGGAACGAGAGCCACAGAGAC3', LWR 5'CATTAAGGGA GCCTTCCGTTC3', PRB FAM-5'CGGAAGGTCGACCCTAATCTCATGGAAA3'-TAMRA; CD34-specific for both mouse splice variants, UPR 5'GAGGCTG ATGCTGGTGCTAGT3'; LWR 5'CAGCAAACACTCAGGCCTAACC3'; PRB FAM-5'CTGCTCCCTGCTTCTAGCCCAGTCTGA3'-TAMRA; Tie2-specific for mouse mRNA, UPR 5'ATGCCTCTGCTCTCAAGGATG3'; LWR 5'TCTGGCAAATCCTCTATCTGTG G3'; PRB FAM-5'TGAGAAAGAAGGCAGGCCAAGGATGACT3'-BHQ1. The TaqMan RT-PCR reactions were carried out with an ABI PRISM 7700 Sequence Detector (Software: Sequence Detection System v1.6.3 (ABI)) using a standard protocol for RT-PCR (48°C 30min, 95°C 10min, 40x (95°C 15s, 60°C 1min)) with primers at a concentration of 300nM and 100nM for the probe. TaqMan data were calculated by using the Comparative C_{T} method. Here the amount of target mRNA (CD31 or Tie2), normalized to an endogenous reference (CD34) and relative to a calibrator (sucrose group) is given by the formula 2^{-ΔΔC}T. Data for individual mice are shown as C_{T} of CD31, CD34 or Tie2 mRNA relative to sucrose presenting the mean of a triplicate ± s.e.m.. Data for treatment groups are shown as ΔΔC_{T} normalized to an endogenous reference and relative to sucrose presenting the mean of 6 - 8 mice per group ± s.e.m..

### Quantification of soluble Tie2 by ELISA

For serum analysis blood was collected from anesthetized mice by orbital sinus bleeding on day 0 and day 5. Soluble Tie2 was measured by ELISA (R&D Systems, Minneapolis, USA) according to the manufacturer's instructions.

### Quantification of Interleukin-12 by ELISA

Male C57BL/6 mice received a single 200µl tail-vein injection of Poly(I:C)- (Sigma, Taufkirchen, Germany) or siRNA-lipoplex solution (final dose of 1.88 mg/kg siRNA or Poly(I:C) and 14.5 mg/kg lipid). Blood was harvested from anesthetized mice by orbital sinus bleeding 2 and 24 hours post injection and serum IL-12 (p40) as well as interferon-α levels were measured by ELISA (R&D Systems, Minneapolis, USA) according to the manufacturer's instructions.

### Mouse studies

Immune deficient male Hsd:NMRI-nu/nu nude mice (9 weeks) were used for toxicity assessment of siRNA-lipoplexes *in vivo* as well as for detection of RNAi (knock-down analysis *in vivo*) and Tie2 ELISA. The microscopic analysis of organ and cell type distribution of fluorescently labelled siRNA-lipoplexes, and the IL-12 ELISA analysis were carried out with immune competent male C57BL/6 mice (8-10 weeks). The animal maintenance and experiments were conducted according to the approved protocols and in compliance with the guidelines of the Landesamt für Arbeits-, Gesundheitsschutz und technische Sicherheit Berlin, Germany (No. G0264/99).

### Statistical analysis

Data are expressed as means ± s.e.m. Statistical significance of differences was determined by the Mann-Whitney U test. P values < 0.05 were considered statistically significant.

### Example 2: Characterization of siRNA-lipoplexes in vitro

We have employed 19-mer siRNA duplexes lacking 3'-overhangs, which are chemically stabilized by alternating 2'-O-methyl sugar modifications on both strands (Czauderna et al., 2003), whereby unmodified nucleotides face modified ones on the opposite strand as depicted in Table 1 of Example 1.

It has been demonstrated previously in cell culture (ex vivo) (Czauderna et al., 2003) that these particularly modified molecules enhances resistance towards serum nuclease while RNAi activity is preserved. First we analyzed whether these molecules mediate RNAi in cell culture either complexed with cationic liposomes or without formulation ("naked"). For this purpose we synthesized a newly designed cationic lipid, referred to as AtuFECT01, in combination with commercially available helper lipids the structure of which are depicted in Fig. 1a. This novel lipid is characterized by a highly charged head group, which allows for more efficient siRNA-binding as compared to other commercially available cationic lipids such as DOTAP or DOTMA. In the following study we used siRNA-lipoplexes consisting of positively charged liposomes (50 mol% cationic lipid AtuFECT01, 49 mol% neutral/helper lipid DPhyPE, and 1 mol% DSPE-PEG) in combination with different target specific siRNA molecules. The liposomes and the siRNA-lipoplexes were characterized regarding seize and charge by QELS (unimodal analysis at an angle of 90°) and zeta-potential measurement. The results thereof are depicted in Figs. 1c and 1d. The zeta potential of a representative cationic lipid formulation was +63mV, while the lipoplex formulation in accordance with the present invention showed a zeta potential of +46mV.

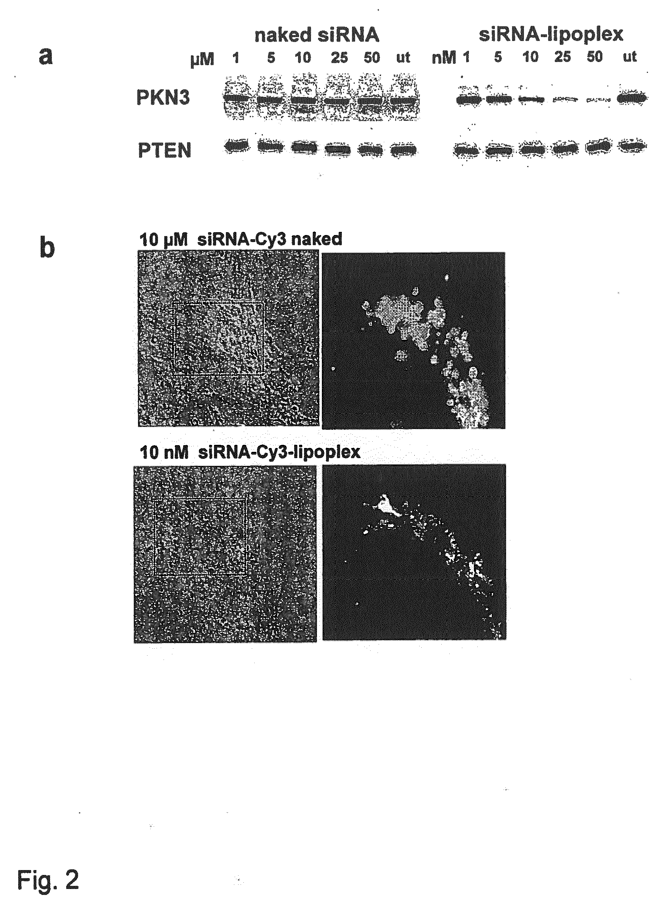

Immunoblot analysis performed for in vitro delivery demonstrated that no gene silencing occurred when naked siRNA was applied at even micromolar concentrations compared to nanomolar concentrations used for siRNA-lipoplexes as may be taken from Fig. 2a. To analyze whether the lack of gene silencing was the result of an inefficient cellular uptake due to repulsive effects between the anionic siRNAs and the negatively charged cell membrane, we employed 3' fluorescently (Cy3) labeled siRNAs to study their uptake by confocal microscopy. We, and others have previously shown that fluorescence labeling at the 3'-end of the antisense molecule does not impair RNA silencing activity when transfected with delivery vehicles (Chiu and Rana, 2002; Czauderna et al., 2003). Surprisingly, we observed a significant uptake of fluorescently labeled siRNAs in the absence of transfection reagents when high concentrations (10µM) of siRNA-Cy3 molecules were applied, as depicted in Fig. 2b. In contrast, equivalent siRNA-Cy3 uptake was achieved when transfected with AtuFECT01 at a thousand fold lower siRNA-Cy3 concentration (10nM). These results indicate that siRNA-lipoplexes provide two beneficial effects for functional delivery of siRNAs: an improved cellular uptake and more importantly, the escape from the endocytotic/endosomal pathway into the cytoplasm (Zelphati and Szoka, 1996), where RNAi-mediated mRNA degradation takes place.

### Example 3: PEGylated siRNA-lipoplexes are functional in vitro and suitable for in vivo application

It has been suggested that cationic liposomal particles can interact with negatively charged serum proteins or bind to other serum components. These unspecific interactions might negatively influence the distribution and delivery properties of the liposomal formulations *in vivo.* To overcome this problem, many liposomal carriers are coated with the polymer poly(ethylene glycol), PEG, to avoid carrier clearance by serum proteins or complement system and improve circulation time. In addition, the incorporation of PEG may help to stabilize the liposomes by shielding and reduce macrophage clearance (Allen et al., 1995; Felgner et al., 1987).

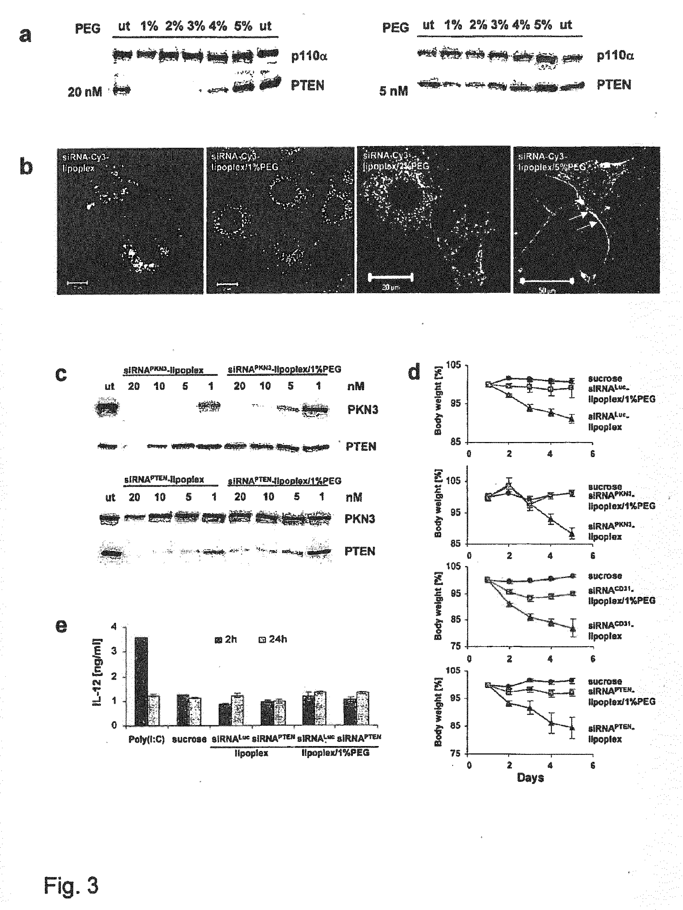

In order to demonstrate the advantage of PEGylation in the case of siRNA-lipoplexes we performed experiments with and without PEGylated lipid. In the following experiments we used siRNA-lipoplexes comprising of positively charged liposomes (cationic lipid Atufect01, neutral/helper lipid DPhyPE, and different amounts of DSPE-PEG-2000) in combination with different target specific siRNA molecules. First, we determined the effect of different amounts of PEGylation on RNA interference activity *in vitro.* SiRNA mediated gene silencing was completely abolished in the presence of 5 mol% of DSPE-PEG-2000 but was maintained in the presence of 1-2 mol% in the formulation. The results are depicted in Fig. 3a which shows the result of a Western blot analysis testing of siRNA^{PTEN} liposomal formulations with different mol% of PEG by transfection of HeLa cells (20 nM (left panel) or 5 nM (right panel) siRNA concentration; ut: untreated); whereby protein extracts were probed with anti anti-PTEN and anti-p110α. The intracellular distribution of fluorescently labelled siRNA lipoplexes changed upon 1-2% PEGylation from large interconnected perinuclear vesicles to small uniform vesicles as depicted in Fig. 3b. In contrast, at 5 mol% of PEGylation, when no RNAi was observed (Fig. 3a), cellular uptake appeared to be blocked, since lipoplexed siRNA-Cy3 mainly became attached to the cell's surface as may be taken from Fig. 3b, right panel. When siRNA complementary to the target sequence of the PTEN or PKN3 gene was formulated in the presence or absence of 1 mol% DSPE-PEG-2000, a specific siRNA mediated protein knockdown was observed regardless of the PEGylation as may be taken from Fig. 3c. However, non-PEGylated siRNA-lipoplexes gave rise to a slightly more efficient protein knockdown at lower concentrations when compared to PEGylated variants (Figure 3c, compare 1nM siRNA concentrations), but exhibited also unspecific knockdown effects (see PTEN loading control for siRNA^{PKN3}-lipoplexes) when applied at higher doses (20 nM, Fig. 3c). This unspecific inhibition of a non-target protein level is probably due to a more pronounced toxic effect of the non-PEGylated lipoplex *in vitro.*

To analyze whether the PEGylation of the siRNA-lipoplexes can also reduce the toxicity *in vivo* we applied identical doses of PEGylated (1 mol% DSPE-PEG-2000) and non-PEGylated siRNA-lipoplexes by tail-vein injection into mice. Consecutive daily treatments (day 1 to 5) of non-PEGylated siRNA-lipoplexes (four different siRNA sequences were used siRNA^{Luc}, siRNA^{PKN3}, siRNA^{CD31} and siRNA^{PTEN}) by systemic administration (i.v.) caused loss in body weight over time, while mice treated with the same daily doses of PEGylated variants (1 mol% DSPE-PEG-2000) appeared unaffected as may be taken from Fig. 3d.

To elucidate the differences in body weight loss after treatment with PEGylated and non-PEGylated siRNA-lipoplexes, a possible immune reaction upon lipoplex treatment was analyzed. For this reason, interleukin-12 level (IL-12) was assayed (Alexopoulou et al., 2001; Liu et al., 2003) in immune competent mice after single i.v. bolus of non complexed Poly(I:C) (positive control) or PEGylated and non-PEGylated siRNA-lipoplexes (siRNA^{PTEN}, siRNA^{Luc}). The ELISA analysis revealed that no increase of IL-12 occurred upon siRNA-lipoplex treatment regardless of PEGylation as may be taken from Fig. 3e. Therefore, it seems unlikely that an unspecific cytokine response caused the observed body weight reduction upon non-PEGylated siRNA-lipoplex treatment. Taken together these data show that 1 mol% of DSPE-PEG-2000 in the siRNA-lipoplex formulations is sufficient to reduce unspecific toxic side effects *in vivo* without a severe loss in RNAi efficacy *in vitro.* Consequently, a defined degree of PEGylation appears to be an important prerequisite for a safe and efficient *in vivo* application of the here characterized siRNA-lipoplexes.

### Example 4: Specific uptake of lipoplexed siRNA into the vascular endothelium and renal excretion of naked siRNA

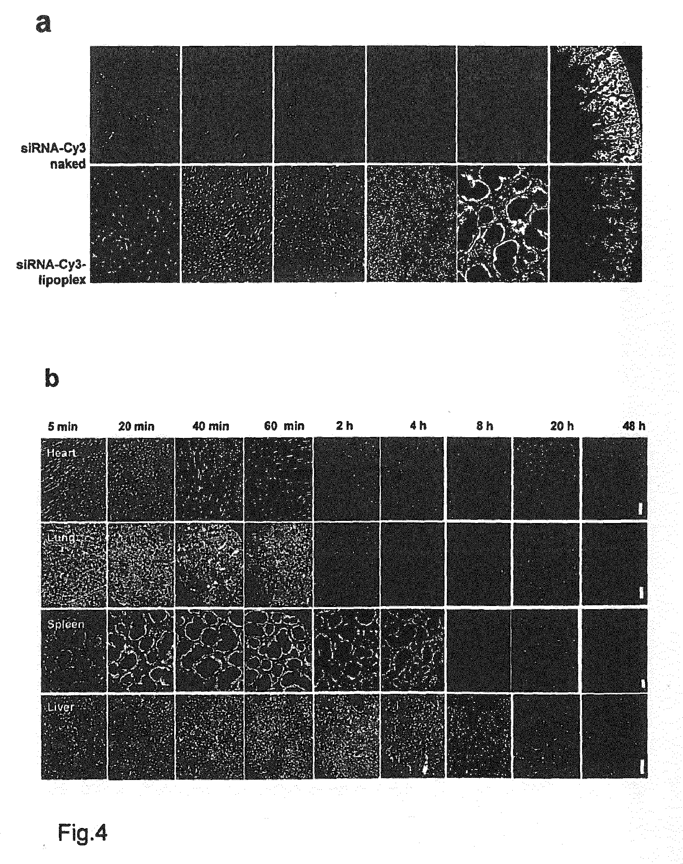

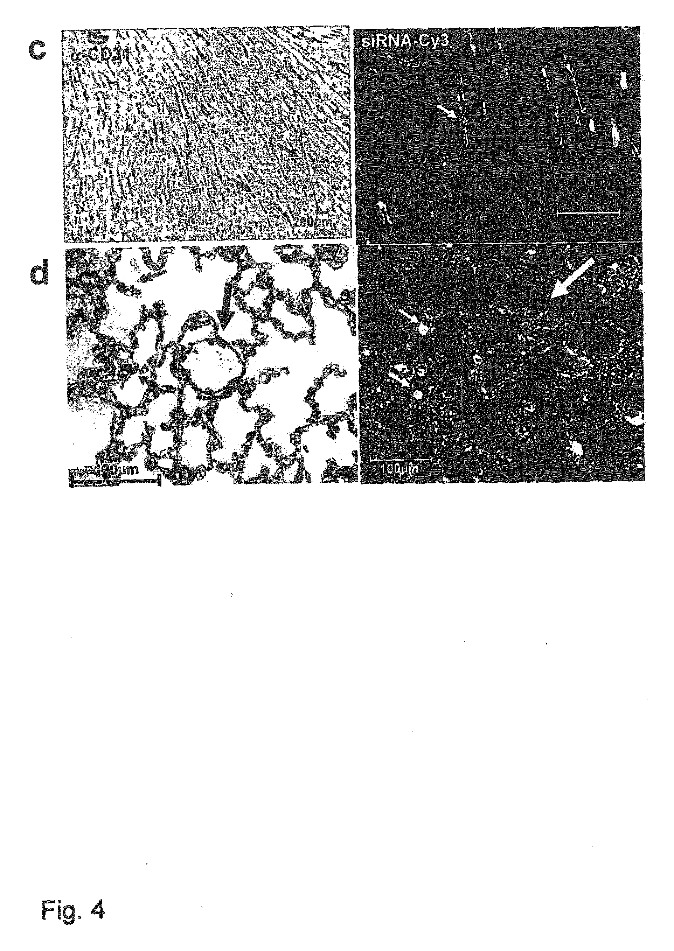

In a next step we set out to investigate the biodistribution and kinetics of siRNA-lipoplexes in comparison to non-formulated siRNA after systemic treatment of mice. For this purpose, we injected a single dose of Cy3 fluorescently labeled siRNA either complexed with lipids (200µl i.v. injection at a final dose of 1.88 mg/kg siRNA-Cy3 and 14.5 mg/kg lipid) or not formulated (siRNA-Cy3: 0.188 mg/ml equal to 15µM) into immune competent mice, and dissected six different organs at nine time points (from 5 min to 48 h) for examination by epifluoresecence and confocal miscroscopy. An initial microscopic analysis revealed that fluorescence was detectable in all tissues analyzed from animals 20 min post treatment with siRNA-Cy3-lipoplex as depicted in Fig.4 a, lower panels. The Cy3-fluorescence appeared in a distinct staining pattern for each organ. This organ-specific Cy3-staining pattern is reminiscent of the tissue endothelia distribution. In contrast, naked siRNA was predominantly found in the kidney 20 min post injection, with no detectable signals in other organs, suggesting a rapid renal excretion of non-formulated siRNA molecules (Fig. 4a, upper row). In addition the applied, naked siRNA-Cy3 accumulates in the pole and lumen of the proximal tubules and in the urine 5 minutes after injection, which was not observed for lipoplexed siRNA-Cy3 . In conclusion, non-formulated siRNAs were not targeted any cell type of analyzed tissues *in vivo* after systemic administration, this being most likely due to instant renal excretion. For the siRNA-Cy3-lipoplexes, the microscopic fluorescence data suggest however, that the siRNA molecules were taken up by the vasculature endothelium in different organs with a profound delayed clearance rate. To analyze the pharmacokinetics of siRNA-Cy3-lipoplexes in more detail we compared the uptake of siRNA-Cy3-lipoplexes more closely at different time points. The four organs (lung, heart, liver, and spleen) with the highest amount of Cy3-fluoresecence at 20 min post injection (200µl i.v. injection at a final dose of 1.88 mg/kg siRNA-Cy3 and 14.5 mg/kg lipid) were selected for this analysis. The Cy3-fluorescence was compared by microscopic examination using identical recording parameters. Substantial amounts of lipoplexed siRNA-Cy3 accumulated in all of these organs starting five minutes after injection as may be taken from Fig. 4b. This level of fluorescence declined gradually within the following 2 hour period in heart and lung tissue. In contrast, most fluorescence was detected in the spleen 20 min after siRNA-Cy3-lipoplex administration, and was retained up to 20 hours in this tissue. In liver, siRNA-Cy3-lipoplex accumulated over time resulting in the highest amount of Cy3-fluorescence at 2 hours post injection, before tapering off during the next 4-20 h time period. Remarkably, the distinct fluorescence staining pattern of the siRNA-Cy3-lipoplex in each tissue did not change over time suggesting that the siRNA molecules do not diffuse throughout the entire tissue. No Cy3-fluorescence was observed at 48 h post single administration of siRNA-Cy3-lipoplexes in any of the samples analyzed by this microscopic assay. These results indicate that siRNA molecules formulated in lipoplexes attain a better organ uptake compared to those administered as naked siRNAs.

The improved siRNA uptake of an organ, however, does not necessarily indicate an intracellular or cell type specific uptake of these molecules, which is a prerequisite for the functionality of the delivered siRNAs. A more detailed analysis of formulated siRNA-Cy3 uptake in the heart and lung by confocal microscopy revealed that on the cellular level, fluorescence staining was predominantly present in the linings of the blood vessels suggesting delivery to endothelial cells. The vascular endothelium in the heart was visualized by immunohistochemistry using an anti-CD31 antibody. The results are depicted in Fig. 4c. The staining for the CD31 expressing endothelial cells illustrates the presence of numerous blood capillaries along the cardiac muscle cells (Fig. 4c; arrow: cross and longitudinal sections of capillaries). These capillary structures were decorated in the heart from mice treated with a single i.v. injection of siRNA-Cy3-lipoplex as revealed by confocal microscopy (Fig. 4c, right panel).

Confocal microscopy of lung sections from mice treated with siRNA-Cy3-lipoplexes revealed a punctuate staining of the alveolar wall, but not of the bronchiole epithelium as may be taken from Fig. 4d. The alveolar wall is traversed by the endothelium of the alveolar capillaries as visualized by staining with anti-CD31 (Fig. 4D, left panel). We therefore conclude that siRNAs lipoplexed using the here described liposomes become delivered to the capillary endothelium of the lung. A similar endothelial cell specific siRNA uptake was observed for other organs including liver, pancreas, kidney, small intestine, stomach. Taken together, these data demonstrate that cationic lipid based formulations of siRNAs improve the biodistribution properties of siRNAs and allow for a predominant uptake of siRNAs into endothelial cells throughout the body.

### Example 5: siRNA-lipoplex mediated RNAi in the vasculature of lung, heart and liver

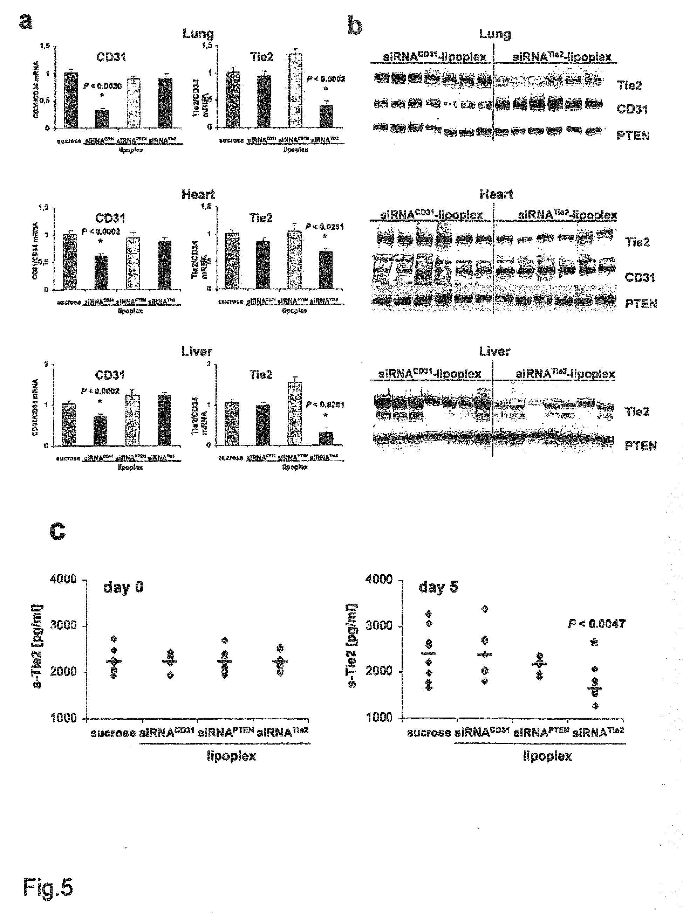

Systemic treatment of mice with siRNA-Cy3-lipoplex suggests a delivery of siRNAs to the endothelial compartment of different organs. Following this observation, we then aimed to correlate siRNA uptake and distribution with the efficacy of RNA interference in particular organs. For this purpose, we designed the following *in vivo* experiment: Nude mice (6-8 per cohort) were treated with four consecutive daily i.v. injections (daily dose: 1.88 mg/kg siRNA and 14.5 mg/kg lipid) with three target specific siRNA-lipoplexes. Potent siRNAs specific for the two endogenous gene targets, CD31 (PECAM-1) and Tie2, were identified in vitro and applied to demonstrate RNAi silencing in the vasculature of selected organs. Importantly, the expression of these two genes is highly restricted to endothelial cells. Additional groups of mice were treated in parallel with sucrose solution or with siRNA-lipoplex specific for the murine PTEN coding sequence to control for unspecific effects. PTEN is, in contrast to CD31 and Tie2, ubiquitously expressed in all cell types of the mice. Gene expression was assessed by measuring changes in mRNA levels employing RT-PCR and protein levels by immunoblot and ELISA methods 24 h after the last siRNA-lipoplex treatment. The results are depicted in Fig. 5. Total RNA was prepared from lung, heart and liver of corresponding treatment groups (sucrose, siRNA^{PTEN}, siRNA^{Tie2}, siRNA^{CD31}) to analyze mRNA knockdown in the endothelium of the two target genes by quantitative RT-PCR (TaqMan). The mRNA level of CD34, another gene with a restricted expression to vascular endothelial cells was measured to normalize for equivalent amounts of RNA from endothelial cells. The mean ratio of CD31 or Tie2 mRNA level normalized to CD34 mRNA level is shown in Fig. 5a. Only in samples derived from animals treated with siRNA^{Tie2}- and siRNA^{cD31}-lipoplexes, Tie2 and CD31 mRNA levels were significantly reduced, as revealed by the respective mRNA quantification data. The reduction in Tie2 mRNA levels in the siRNA^{Tie2}-lipoplex treatment group or CD31 mRNA for the siRNA^{CD31}-lipoplex group demonstrates the target specificity of the siRNA treatment. In the liver the knockdown in mRNA level was more prominent for Tie2 (Fig. 5a, lower panel), whereas in lung and heart the expression of both target genes were inhibited similarly. To corroborate the siRNA mediated inhibition of the both target genes we set out to confirm the inhibition of Tie2 protein expression by immunoblot (Fig. 5b). Organ protein lysates from 6-7 individual animals of the siRNA^{Tie2}-lipoplex or the siRNA^{CD31}-lipoplex treatment groups were prepared, separated by SDS-polyacrylamide gelelectrophoresis, and corresponding immunoblots were probed with anti-Tie2 and anti-PTEN (loading control). A significant reduction of Tie2 protein was observed in all three organs from siRNA^{Tie2}-lipoplex treated animals. With protein lysates from liver we were not able to detect significant amounts of CD31 by immunoblot. However, a significant siRNA^{CD31}-lipoplex specific reduction of CD31 protein level was demonstrated for lung and heart. Taken together, these data demonstrate an siRNA mediated "gene silencing" on mRNA and protein level in the vascular endothelium of the mouse.

Since the Tie-2 protein level was significantly reduced in protein lysates of all three analysed organs we analysed whether we can detect a reduction in the level of the soluble form of Tie2 in the blood of siRNA^{Tie2}-lipoplex treated animals. The Tie2 protein acts as a receptor tyrosine kinase exclusively in endothelial cells in concert with its ligands, the angiopoietins, thus contributing to vessel remodelling and integrity (Davis et al., 1996; Thurston, 2003). The soluble form of Tie2, s-Tie2, is a product of proteolytic cleavage of the receptor's extracellular domain which can be easily detected in the blood by ELISA assays. We monitored changes in s-Tie2 in the serum before and after siRNA-lipoplex treatment using an s-Tie2 ELISA as readout. All four groups of mice tested showed similar levels in s-Tie2 before treatment (day 0; Fig. 5c, left diagram). Mice treated with siRNA^{Tie2}-lipoplex exhibited a significant reduction in s-Tie2 levels when compared to treated mice from control cohorts (sucrose, siRNA^{PTEN}, siRNA^{CD31}) on day 5 of the treatment schedule (day 5; Fig. 5c, right diagram). This result implies that systemic siRNA^{Tie2}-lipoplex treatment affects overall Tie2 gene expression *in vivo*, presumably by suppressing Tie2 protein expression in the body's vasculature endothelium. In conclusion, AtuFECT01-based siRNA-lipoplexes are targeted to the vascular endothelium of many tissues after i.v. administration. Repeated administration of siRNA-lipoplexes resulted in RNA as well as protein knockdown of endothelial gene expression in a target specific manner in tissues such as lung, heart, and liver.

### Example 6: Method for the preparation of siRNA lipoplexes

### siRNA lipoplexes having a mean particle size of about 120 nm

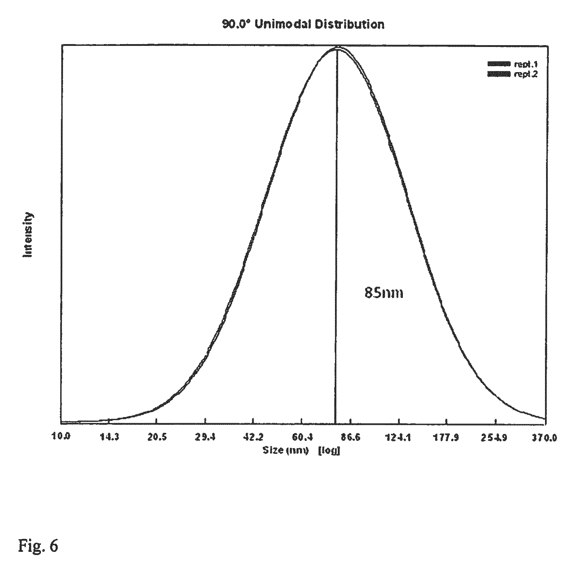

Solutions of the lipids forming the lipoplexes of the present application in chloroform (c = 20 mg/ml) were dispensed into a 100 ml round bottom flask so that in the mixture a ratio of cationic lipid β-(L-arginyl)-2,3-L-diaminopropionic acid-*N*-palmityl-*N*-oleyl-amide tri-hydrochloride (AtuFECT01) : helper lipid 1,2-diphytanoyl-sn-glycero-3-phosphoethanolamine (DPhyPE) : PEG lipid N-(Carbonyl-methoxypolyethyleneglycol-2000)-1,2-distearoyl-sn-glycero-3-phosphoethanolamine sodium salt (DSPE-PEG) of 50 mol%:49 mol%:1 mol% is resulting. Subsequently, the solvent is removed under vacuum and the resulting lipid film is dried under high vacuum for 4 hours. To the dried lipids, a 270 mM sterile sucrose solution is added, resulting in a concentration of 4.335 mg/ml total lipid. By short sonification in an ultrasound bath for 5 minutes, the lipids are dispersed and subsequently homogenised by high pressure homogenisation (Avestin C3). Such homogenisation of the liposomes is performed by subjecting them to 21 cycles at 750 bar and 52 cycles at 1250 bar. The thus obtained liposomes have a mean particle size of about 85 nm as depicted in Fig. 6 and as determined by QELS (Beckman-Coulter N5 and Malvern Zetasizer NS).

These liposomes are subject to a further treatment under aseptic conditions. For such purpose, they are mixed with the same volume of an siRNA solution in 270 mM sucrose (c = 0.5625 mg/ml). The siRNA solution is added to the liposomes under agitation at 1500 rpm by means of a syringe. This results in the formation of lipoplexes having a mean particle size of about 120 nm.

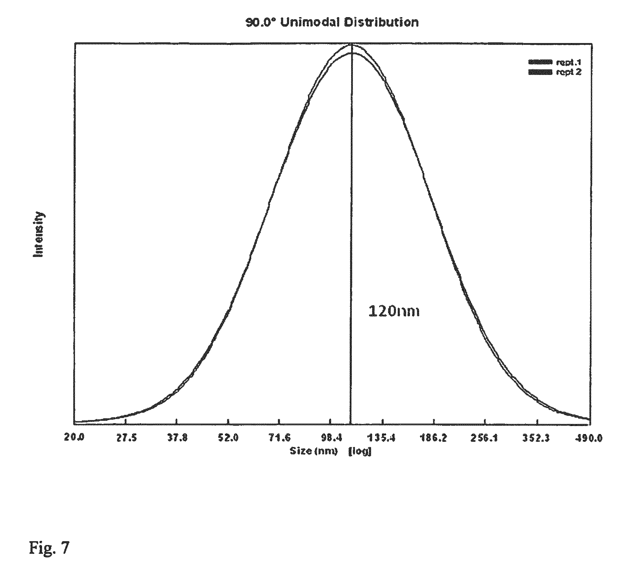

The size distribution of the thus obtained lipoplexes is illustrated in Fig. 7. The particle size was determined by QELS (Beckman-Coulter N5 and Malvern Zetasizer NS).

### siRNA lipoplexes having a mean particle size of about 60 nm

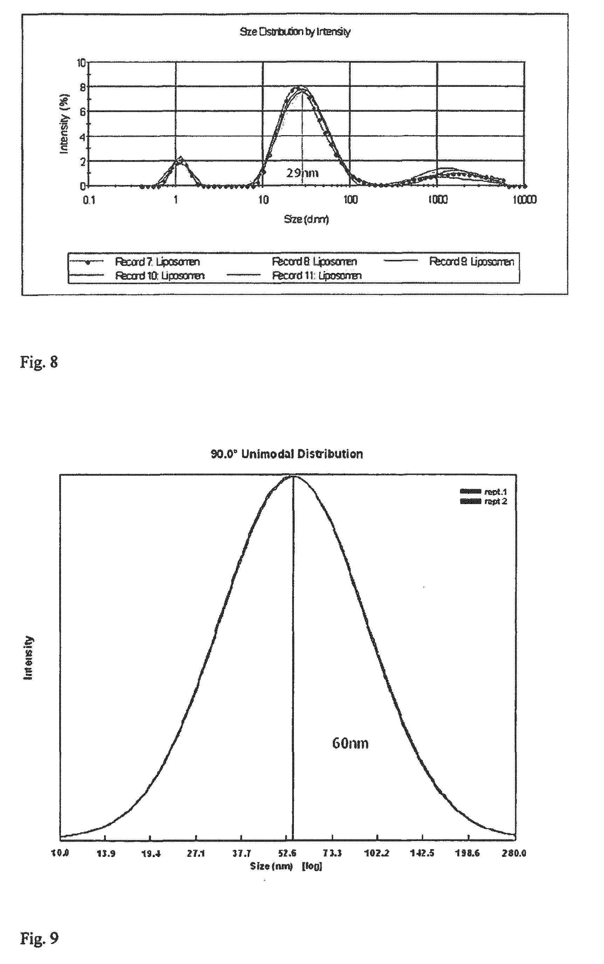

1 ml of a solution containing the lipids as specified above in connection with the preparation of siRNA lipoplexes having a mean particle size of about 120 nm (c = 86.5 mg/ml overall lipid in the above ratio of 50:49:1) in 30 % tert.-butanol is added by means of a syringe under aseptic conditions and agitation at 1500 rpm to 19 ml of a sterile 270 mM sucrose solution within one minute. By doing so, liposomes having a mean particle size of about 30 nm may be obtained. Fig. 8 shows the respective size distribution. The particle size was determined by QELS (Beckman-Coulter N5 and Malvern Zetasizer NS).

The liposome solution thus obtained is aliquoted into 1.6 ml portions in 5 ml-Lyo-Vials which are subsequently shock frozen to -80° C and then lyophilised.

For the preparation of lipoplexes, 3.2 ml of a solution of 0.28 mg/ml siRNA in 135 mM sterile sucrose solution are injected into the Vial containing the lyphilised liposomes which are also referred to as the lyophilisate. The Vial containing the lyophilisate and the solution is subsequently shaken until complete dissolution of the cake formed by the lyophilisate. The thus obtained lipoplexes have a mean particle size of about 60 nm as depicted in Fig. 9. The particle size was determined by QELS (Beckman-Coulter N5 and Malvern Zetasizer NS).

### References

To the extent it is referred herein to various documents of the prior art, such documents the complete bibliographic data of which read as follows, are incorporated herein in their entirety by reference.
Alexopoulou, L., Holt, A.C., Medzhitov, R. and Flavell, R.A. (2001) Recognition of double-stranded RNA and activation of NF-kappaB by Toll-like receptor 3. Nature, 413, 732-738.
Allen, T.M., Hansen, C.B. and de Menezes, D.E.L. (1995) Pharmacokinetics of long-circulating liposomes. Advanced Drug Delivery Reviews, 16,267-284.
Barron, L.G., Uyechi, L.S. and Szoka, F.C., Jr. (1999) Cationic lipids are essential for gene delivery mediated by intravenous administration of lipoplexes. Gene Ther, 6, 1179-1183.
Braasch, D.A., Paroo, Z., Constantinescu, A., Ren, G., Oz, O.K., Mason, R.P. and Corey, D.R. (2004) Biodistribution of phosphodiester and phosphorothioate siRNA. Bioorg Med Chem Lett, 14, 1139-1143.
Chae, S.S., Paik, J.H., Furneaux, H. and Hla, T. (2004) Requirement for sphingosine 1-phosphate receptor-1 in tumor angiogenesis demonstrated by in vivo RNA interference. J Clin Invest, 114, 1082-1089.
Chien, P.Y., Wang, J., Carbonaro, D., Lei, S., Miller, B., Sheikh, S., Ali, S.M., Ahmad, M.U. and Ahmad, I. (2005) Novel cationic cardiolipin analogue-based liposome for efficient DNA and small interfering RNA delivery in vitro and in vivo. Cancer Gene Ther, 12, 321-328.
Chiu, Y.L. and Rana, T.M. (2002) RNAi in human cells: basic structural and functional features of small interfering RNA. Mol Cell, 10, 549-561.
Chiu, Y.L. and Rana, T.M. (2003) siRNA function in RNAi: a chemical modification analysis. Rna, 9, 1034-1048.
Czauderna, F., Fechtner, M., Dames, S., Aygun, H., Klippel, A., Pronk, G.J., Giese, K. and Kaufmann, J. (2003) Structural variations and stabilising modifications of synthetic siRNAs in mammalian cells. Nucleic Acids Res, 31, 2705-2716.
Davis, S., Aldrich, T.H., Jones, P.F., Acheson, A., Compton, D.L., Jain, V., Ryan, T.E., Bruno, J., Radziejewski, C., Maisonpierre, P.C. and Yancopoulos, G.D. (1996) Isolation of angiopoietin-1, a ligand for the TIE2 receptor, by secretion-trap expression cloning. Cell, 87, 1161-1169.
Felgner, P.L., Gadek, T.R., Holm, M., Roman, R., Chan, H.W., Wenz, M., Northrop, J.P., Ringold, G.M. and Danielsen, M. (1987) Lipofection: a highly efficient, lipid-mediated DNA-transfection procedure. Proc Natl Acad Sci USA, 84, 7413-7417.
Hu-Lieskovan, S., Heidel, J.D., Bartlett, D.W., Davis, M.E. and Triche, T.J. (2005) Sequence-Specific Knockdown of EWS-FLI1 by Targeted, Nonviral Delivery of Small Interfering RNA Inhibits Tumor Growth in a Murine Model of Metastatic Ewing's Sarcoma. Cancer Res, 65, 8984-8992.
Klippel, A., Escobedo, J.A., Hirano, M. and Williams, L.T. (1994) The interaction of small domains between the subunits of phosphatidylinositol 3-kinase determines enzyme activity. Mol Cell Biol, 14,2675-2685.
Klippel, A., Escobedo, M.A., Wachowicz, M.S., Apell, G., Brown, T.W., Giedlin, M.A., Kavanaugh, W.M. and Williams, L.T. (1998) Activation of phosphatidylinositol 3-kinase is sufficient for cell cycle entry and promotes cellular changes characteristic of oncogenic transformation. Mol Cell Biol, 18, 5699-5711.
Landen, C.N., Jr., Chavez-Reyes, A., Bucana, C., Schmandt, R., Deavers, M.T., Lopez-Berestein, G. and Sood, A.K. (2005) Therapeutic EphA2 gene targeting in vivo using neutral liposomal small interfering RNA delivery. Cancer Res, 65,6910-6918.
Leenders, F., Mopert, K., Schmiedeknecht, A., Santel, A., Czauderna, F., Aleku, M., Penschuck, S., Dames, S., Sternberger, M., Rohl, T., Wellmann, A., Arnold, W., Giese, K., Kaufmann, J. and Klippel, A. (2004) PKN3 is required for malignant prostate cell growth downstream of activated PI 3-kinase. Embo J, 23, 3303-3313.
Liu, L., Zhou, X., Shi, J., Xie, X. and Yuan, Z. (2003) Toll-like receptor-9 induced by physical trauma mediates release of cytokines following exposure to CpG motif in mouse skin. Immunology, 110, 341-347.
Liu, T.G., Yin, J.Q., Shang, B.Y., Min, Z., He, H.W., Jiang, J.M., Chen, F., Zhen, Y.S. and Shao, R.G. (2004) Silencing of hdm2 oncogene by siRNA inhibits p53-dependent human breast cancer. Cancer Gene Ther, 11, 748-756.
Morrissey, D.V., Lockridge, J.A., Shaw, L., Blanchard, K., Jensen, K., Breen, W., Hartsough, K., Machemer, L., Radka, S., Jadhav, V., Vaish, N., Zinnen, S., Vargeese, C., Bowman, K., Shaffer, C.S., Jeffs, L.B., Judge, A., MacLachlan, I. and Polisky, B. (2005) Potent and persistent in vivo anti-HBV activity of chemically modified siRNAs. Nat Biotechnol, 23, 1002-1007.
Muratovska, A. and Eccles, M.R. (2004) Conjugate for efficient delivery of short interfering RNA (siRNA) into mammalian cells. FEBS Lett, 558, 63-68.
Nogawa, M., Yuasa, T., Kimura, S., Tanaka, M., Kuroda, J., Sato, K., Yokota, A., Segawa, H., Toda, Y., Kageyama, S., Yoshiki, T., Okada, Y. and Maekawa, T. (2005) Intravesical administration of small interfering RNA targeting PLK-1 successfully prevents the growth of bladder cancer. J Clin Invest, 115, 978-985.
Richard, J.P., Melikov, K., Vives, E., Ramos, C., Verbeure, B., Gait, M.J., Chernomordik, L.V. and Lebleu, B. (2003) Cell-penetrating peptides. A reevaluation of the mechanism of cellular uptake. J Biol Chem, 278, 585-590.
Schiffelers, R.M., Ansari, A., Xu, J., Zhou, Q., Tang, Q., Storm, G., Molema, G., Lu, P.Y., Scaria, P.V. and Woodle, M.C. (2004) Cancer siRNA therapy by tumor selective delivery with ligand-targeted sterically stabilized nanoparticle. Nucleic Acids Res, 32, e149.
Shadidi, M. and Sioud, M. (2003) Identification of novel carrier peptides for the specific delivery of therapeutics into cancer cells. Faseb J, 17, 256-258.
Song, E., Zhu, P., Lee, S.K., Chowdhury, D., Kussman, S., Dykxhoorn, D.M., Feng, Y., Palliser, D., Weiner, D.B., Shankar, P., Marasco, W.A. and Lieberman, J. (2005) Antibody mediated in vivo delivery of small interfering RNAs via cell-surface receptors. Nat Biotechnol, 23, 709-717.
Soutschek, J., Akinc, A., Bramlage, B., Charisse, K., Constien, R., Donoghue, M., Elbashir, S., Geick, A., Hadwiger, P., Harborth, J., John, M., Kesavan, V., Lavine, G., Pandey, R.K., Racie, T., Rajeev, K.G., Rohl, I., Toudjarska, I., Wang, G., Wuschko, S., Bumcrot, D., Koteliansky, V., Limmer, S., Manoharan, M. and Vornlocher, H.P. (2004) Therapeutic silencing of an endogenous gene by systemic administration of modified siRNAs. Nature, 432, 173-178.
Takeshita, F., Minakuchi, Y., Nagahara, S., Honma, K., Sasaki, H., Hirai, K., Teratani, T., Namatame, N., Yamamoto, Y., Hanai, K., Kato, T., Sano, A. and Ochiya, T. (2005) Efficient delivery of small interfering RNA to bone-metastatic tumors by using atelocollagen in vivo. Proc Natl Acad Sci U S A, 102, 12177-12182.
Thurston, G. (2003) Role of Angiopoietins and Tie receptor tyrosine kinases in angiogenesis and lymphangiogenesis. Cell Tissue Res, 314, 61-68.
Turner, J.J., Arzumanov, A.A. and Gait, M.J. (2005) Synthesis, cellular uptake and HIV-1 Tat-dependent trans-activation inhibition activity of oligonucleotide analogues disulphide-conjugated to cell-penetrating peptides. Nucleic Acids Res, 33, 27-42.
Uprichard, S.L. (2005) The therapeutic potential of RNA interference. FEBS Lett, 579, 5996-6007.
Urban-Klein, B., Werth, S., Abuharbeid, S., Czubayko, F. and Aigner, A. (2005) RNAi-mediated genetargeting through systemic application of polyethylenimine (PEI)-complexed siRNA in vivo. Gene Ther, 12, 461-466.
Yano, J., Hirabayashi, K., Nakagawa, S., Yamaguchi, T., Nogawa, M., Kashimori, I., Naito, H., Kitagawa, H., Ishiyama, K., Ohgi, T. and Irimura, T. (2004) Antitumor activity of small interfering RNA/cationic liposome complex in mouse models of cancer. Clin Cancer Res, 10, 7721-7726.
Zelphati, O. and Szoka, F.C., Jr. (1996) Mechanism of oligonucleotide release from cationic liposomes. Proc Natl Acad Sci USA, 93, 11493-11498.

The features of the present invention disclosed in the specification, the claims and/or the drawings may both separately and in any combination thereof be material for realizing the invention in various forms thereof.

## Claims

1. A lipid composition contained in and/or containing a carrier comprising
at least a first lipid component,
at least a first helper lipid, and
a shielding compound which is optionally removable from the lipid composition under in vivo conditions,
wherein the lipid composition containing carrier has an osmolarity of about 50 to 600 mosmole/kg, preferably about 250 - 350 mosmole/kg, and more preferably about 280 to 320 mosmole/kg, and/or
wherein liposomes formed by the first lipid component and/or one or both of the helper lipid and the shielding compound in the carrier have a particle size of about 20 to 200 nm,
and wherein the shielding compound comprises a pH-sensitive linker or a pH-sensitive moiety.

2. The lipid composition according to claim 1, wherein the shielding compound is selected from the group comprising PEG, HEG, polyhydroxyethyl starch (polyHES), a conjugate of PEG and ceramide and polypropylene.

3. The lipid composition according to claim 1 or 2 wherein the helper lipid is selected from ceramide, phospholipids, steroids, PEG moiety, a HEG moiety, a polyhydroxyethyl starch (polyHES) moiety and a polypropylene moiety.

4. The lipid composition according to any preceding claim, wherein the pH-sensitive linker or pH-sensitive moiety is anionic.

5. The lipid composition according to any preceding claim wherein the ceramide comprises at least one short carbon chain substituent of from 6 to 10 carbon atoms, preferably 8 carbon atoms.

6. The lipid composition according to any preceding claim, wherein the composition comprises a further constituent and/or a second helper lipid.

7. The lipid composition according to claim 6, wherein the first and/or second helper lipid is selected from the group comprising 1,2-diphytanoyl-sn-glycero-3-phosphoethanolamine and 1,2-dioleyl-sn-glycero-3-phosphoethanolamine.

8. The lipid composition according to any preceding claims, wherein the first lipid component is a compound according to formula (I), 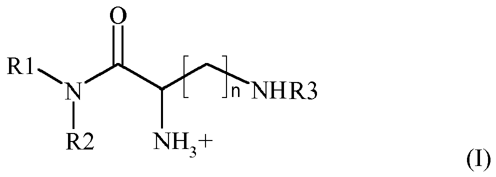 wherein R₁ and R₂ are each and independently selected from the group comprising alkyl;
n is any integer between 1 and 4;
R₃ is an acyl selected from the group comprising lysyl, ornithyl, 2,4-diaminobutyryl, histidyl and an acyl moiety according to formula (II), 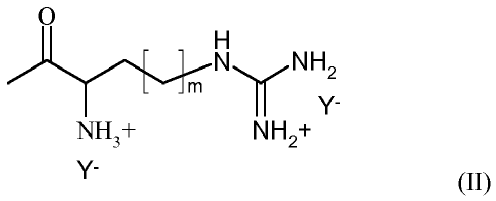 wherein m is any integer from 1 to 3, wherein the NH₃⁺ is optionally absent, and
Y⁻ is a pharmaceutically acceptable anion.

9. The lipid composition according to claim 8, wherein R₁ and R₂ are each and independently selected from the group comprising lauryl, myristyl, palmityl and oleyl.

10. The lipid composition according to claim 8 or 9, wherein R₁ is lauryl and R₂ is myristyl; or
R₁ is palmityl and R₂ is oleyl.

11. The lipid composition according to any one of claims 8 to 10, wherein m is 1 or 2.

12. The lipid composition according to any one of claims 8 to 11, wherein the compound is a cationic lipid, preferably in association with an anion Y⁻.

13. The lipid composition according to any one of claims 8 to 12, wherein Y⁻ is selected from the group comprising halogenids, acetate and trifluoroacetate.

14. The lipid composition according to any one of claims 8 to 13, wherein the compound is selected from the group comprising
- β-arginyl-2,3-diamino propionic acid-N-palmityl-N-oleyl-amide trihydrochloride 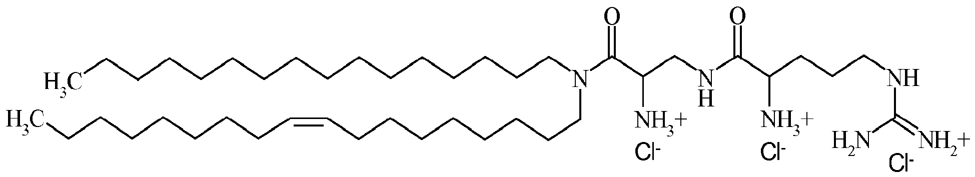
- β-arginyl-2,3-diamino propionic acid-N-lauryl-N-myristyl-amide trihydrochloride 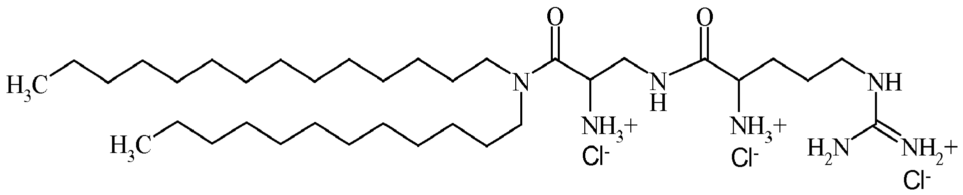 and
- ε-arginyl-lysine-N-lauryl-N-myristyl-amide trihydrochloride

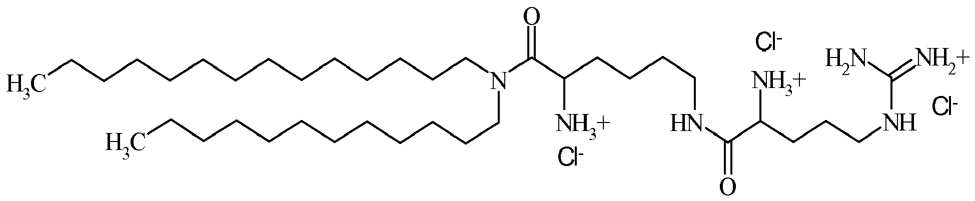

15. The lipid composition according to any preceding claim, wherein the osmolarity is mostly determined by a sugar, whereby said sugar is preferably selected from the group comprising sucrose, trehalose, glucose, galactose, mannose, maltose, lactulose, inulin, raffinose, and any combination thereof, more preferably selected from the group comprising sucrose, trehalose, inulin, raffinose and any combination thereof.

16. The lipid composition according to any preceding claim, wherein the composition contains one or several basic compounds, whereby such basic compounds are preferably selected from the group comprising basic amino acids and weak bases.

17. The lipid composition according to any preceding claim, wherein the lipid composition comprises a nucleic acid, selected from the group comprising RNAi, siRNA, siNA, antisense nucleic acid, ribozymes, aptamers and spiegelmers.

18. The lipid composition according to any of the preceding claims, wherein the composition comprises a nucleic acid and the nucleic acid forms together with the liposome a lipoplex.

19. The lipid composition according to any preceding claim, wherein the concentration of the lipids in the carrier is about from 0.01 to 100 mg/ml, preferably about from 0.01 to 40 mg/ml and more preferably about from 0.01 to 25 mg/ml, each based on the overall amount of lipid provided by the lipoplex.

20. The lipid composition according to any of claims 17 to 19, wherein the nucleic acid is an siRNA and the concentration of the siRNA in the lipid composition is about 0.2 to 0.4 mg/ml, preferably 0.28 mg/ml, and the total lipid concentration is about 1.5 to 2.7 mg/ml, preferably 2.17 mg/ml.

21. The lipid composition according to any preceding claim, wherein the content of the helper lipid component is from about 20 mol % to about 80 mol % , preferably from about 35 mol % to about 65 mol %. of the overall lipid content of the composition or of the lipoplex of claim 18.

22. The lipid composition according to any preceding claim, wherein the lipid is β-arginyl-2,3-diamino propionic acid-N-palmityl-N-oleyl-amide trihydrochloride, and the helper lipid is 1,2-diphytanoyl-sn-glycero-3-phosphoethanolamine.

23. The lipid composition according to any preceding claim, wherein the helper lipid is selected from the group comprising 1,2-distearoyl-sn-glycero-3-phosphoethanolamine and 1,2-dialkyl-sn-glycero-3-phosphoethanolamine.

24. The lipid composition according to any preceding claim, wherein the composition comprises a second helper lipid 1,2-distearoyl-sn-glycero-3-phosphoethanolamine-PEG2000.

25. A pharmaceutical composition comprising a composition according to any preceding claim and optionally a pharmaceutically active compound and preferably a pharmaceutically acceptable carrier.

26. The pharmaceutical composition according to claim 25, wherein the pharmaceutically active compound and/or the further constituent is selected from the group comprising peptides, proteins, antibodies, oligonucleotides, polynucleotides, nucleic acids, DNA, RNA, PNA, LNA, RNAi, siRNA, siNA, antisense nucleic acid, ribozymes, aptamers and spiegelmers.

27. Use of a composition according to any preceding claim for the treatment of a disease selected from cancers, angiogenesis dependent diseases, neoplastic diseases, bone cancer, breast cancer, prostate cancer, cancer of the digestive system, colorectal cancer, liver cancer, lung cancer, kidney cancer, urogenital cancer, pancreatic cancer, pituitary cancer, testicular cancer, orbital cancer, head and neck cancer, cancer of the central nervous system and cancer of the respiratory system.

28. Use of a composition according to any one of claims 1 to 26, for the delivery or transfer of a nucleic acid, preferably a functional nucleic acid, to endothelium, preferably vascular endothelium.

29. An in vitro method for transferring a pharmaceutically active compound and/or a further constituent into a cell or across a membrane, preferably a cell membrane, comprising the following steps:
- providing the cell or the membrane;
- providing a composition according to any preceding claim, whereby the composition optionally comprises a pharmaceutically active compound and/or a further constituent; and
- contacting the cell or the membrane with said composition, and
- detecting the pharmaceutically active compound and/or the further constituent in the cell and/or beyond the membrane.
